(19) Europäisches Patentamt
European Patent Office
Office européen des brevets

(11) EP 0 802 195 B1

(12) **EUROPÄISCHE PATENTSCHRIFT**

(45) Veröffentlichungstag und Bekanntmachung des Hinweises auf die Patenterteilung:
**19.11.2003 Patentblatt 2003/47**

(51) Int Cl.[7]: **C07D 243/02**, A61K 31/55

(21) Anmeldenummer: **97105591.8**

(22) Anmeldetag: **04.04.1997**

# (54) Substituierte 2,3-Benzodiazepinderivate, Verfahren und Zwischenprodukte zu ihrer Herstellung und diese enthaltende pharmazeutische Präparate sowie ihre Verwendung

Substituted 2,3-benzodiazepine derivatives, process and intermediates for their preparation and pharmaceutical compositions comprising them as well as their use

Dérivés de 2,3-benzodiazepine, procédé et intermédiaires pour leur obtention, composés pharmaceutiques les contenant et leur utilisation

(84) Benannte Vertragsstaaten:
**AT CH DE FI LI NL SE**
Benannte Erstreckungsstaaten:
**LT LV SI**

(30) Priorität: **04.04.1996 HU 9600871**

(43) Veröffentlichungstag der Anmeldung:
**22.10.1997 Patentblatt 1997/43**

(73) Patentinhaber: **EGIS GYOGYSZERGYAR RT.**
**1106 Budapest (HU)**

(72) Erfinder:
- **Ling, István, Dr.**
  **1141 Budapest (HU)**
- **Gizella, Abrahám**
  **1118 Budapest (HU)**
- **Berzsenyi, Pál, Dr.**
  **1174 Budapest (HU)**
- **Tarnawa, István, Dr.**
  **1147 Budapest (HU)**
- **Solyom, Sándor, Dr.**
  **1144 Budapest (HU)**
- **Andrási, Ferenc, Dr.**
  **1125 Budapest (HU)**
- **Hámori, Tamás Dr.**
  **1031 Budapest (HU)**
- **Csuzdi, Emese**
  **1046 Budapest (HU)**
- **Horváth, Katalin Dr.**
  **1025 Budapest (HU)**
- **Gál, Melinda Dr. .**
  **1142 Budapest (HU)**
- **Moravcsik, Imre**
  **1095 Budapest (HU)**
- **Szöllosy, Márta**
  **1105 Budapest (HU)**

(74) Vertreter: **Beszédes, Stephan G., Dr.**
**Patentanwalt,**
**Münchener Strasse 80a**
**85221 Dachau (DE)**

(56) Entgegenhaltungen:
**EP-A- 0 492 485** **US-A- 4 322 346**

- **CNS DRUG REV. (CDREFB,1080563X);96; VOL.2 (1); PP.91-126, HUNGARIAN ACADEMY SCIENCES;INSTITUTE EXPERIMENTAL MEDICINE; BUDAPEST; H-1450; HUNG. (HU), XP002035188 VIZI E S ET AL: "2,3-Benzodiazepines (GYKI 52466 and analogs): Negative allosteric modulators of AMPA receptors"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft neue substituierte 2,3-Benzodiazepinderivate, ein Verfahren zu ihrer Herstellung und diese Verbindungen enthaltende pharmazeutische Präparate sowie ihre Verwendung zur Herstellung von Arzneimitteln.

In der chemischen Verbindungsgruppe der 2,3-Benzodiazepinderivate sind mehrere biologisch wertvolle und auch therapeutisch verwendbare Verbindungen, welche am Benzolring dimethoxy- oder methylendioxysubstituiert sind, bekannt. 7,8-Di-(methoxy)-Derivate sind zum Beispiel in den HU-PS 155 572, 179 018, 191 702 und 195 788 sowie in der US-PS 4 322 346 beschrieben. Diese Verbindungen zeigen insbesondere eine anxiolytische und/oder antidepressante beziehungsweise positiv inotrope Wirkung. Benzodiazepinderivate, welche an denselben Stellen des Benzolringes einen Methylendioxy-Substituenten aufweisen, sind zum Beispiel in den HU-PS 191 698, 191 702 und 206 719 beziehungsweise in der US-PS 5 459 137 sowie in CNS Drug Reviews 1996, Vol. 2, No. 1, Seiten 91 bis 126 beschrieben. Im Gegensatz zu den 7,8-di-(methoxy)-substituierten Derivaten haben die in den 7- und 8-Stellungen durch eine Methylendioxygruppe substituierten 2,3-Benzodiazepinderivate insbesondere eine spasmolytische, muskelrelaxante und neuroprotektive Wirkung.

Es ist aus dem Schrifttum wohlbekannt, daß die obigen Verbindungen ihre Wirkung durch nicht-kompetitive Hemmung des AMPA-Rezeptors ausüben (siehe zum Beispiel Donevan, S.D. et al.: Neuron 10 [1993], Seiten 51 bis 59; Donevan, S.D. et al.: J. Pharmacol. Exp. Ther. 271 [1994], Seiten 25 bis 29; Tarnawa, I. et al.: Bioorg. Med. Chem. Lett., 3 [1993], Seiten 99 bis 104).

Ferner ist es bekannt, daß im zentralen Nervensystem von Säugern als wichtigster Neurotransmitter die L-Glutaminsäure vorhanden ist. Unter pathologischen Bedingungen wird die Glutaminsäurekonzentration außerhalb der Zellen krankhaft erhöht, was eine akute oder chronische Schädigung der Zellen hervorruft.

Die stimulierenden Aminosäuren üben ihre Wirkung durch Aktivierung der ionotropen (Ionenkanal) beziehungsweise an die G-Proteine gebundenen metabotropen Rezeptoren aus. Die verschiedenen Typen der ionotropen Glutamatrezeptoren werden den zur selektiven Stimulierung derselben geeigneten Agonisten entsprechend genannt. Dementsprechend wird unter NMDA-, AMPA- und Kainat- (früher Quisqualat genannt) -Rezeptoren unterschieden, wobei innerhalb dieser Gruppen weitere Untergruppen vorhanden sind (Ann. Neurosci. 17 [1994], Seite 31).

Es wurde nachgewiesen, daß bei mehreren mit dem Zentralnervensystem im Zusammenhang stehenden akuten und chronischen Krankheiten (zum Beispiel Epilepsie, mit einem spastischen Zustand der Muskeln verbundenen Krankheitsbildern und verschiedenen neurodegenerativen Krankheiten), Glutamatrezeptoren des AMPA-Typs eine wichtige Rolle spielen und durch Hemmung der AMPA-Rezeptoren eine spasmolytische, muskelrelaxante und neuroprotektive Wirkung hervorgerufen werden kann (siehe zum Beispiel Cerebrovasc. Brain Metab. Rev. 6 [1994], Seite 225; Neurology 44 Suppl. 8 [1994], Seite 14; Pharmacol. Exp. Ther. 260 [1992], Seite 742).

Die Hemmung der Aktivierung der AMPA-Rezeptoren kann sowohl mit kompetitiven als auch mit nicht-kompetitiven Antagonisten durchgeführt werden. Die Verwendung von nicht-kompetitiven Antagonisten ist im allgemeinen vorteilhafter als die der kompetitiven Antagonisten, weil die nicht-kompetitiven Antagonisten einen größeren Schutz im Falle der extrem hohen endogenen Konzentration von stimulierenden Aminosäuren gewähren (Epilepsy Res. 15 [1993], Seite 179).

Unter Berücksichtigung der obigen Tatsachen war die Feststellung, daß die durch eine Methylendioxygruppe substituierten 2,3-Benzodiazepinderivate auf Grund ihrer nicht-kompetitiven AMPA-antagonistischen Wirkung spasmolytische, muskelrelaxante und neuroprotektive Wirkungen ausüben und deshalb in der Therapie als Spasmolytica, Antiepileptica, bei akuten und chronischen neurodegenerativen Krankheitsbildern und im Prinzip bei sämtlichen solchen Krankheiten eingesetzt werden können, bei welchen die Hemmung der stimulierenden Aminosäure auf dem Rezeptorniveau wünschenswert ist, von sehr großer Bedeutung.

Der Erfindung liegt die Aufgabe zugrunde, überlegene pharmakologische Wirkungen, insbesondere zur Behandlung von mit einem spastischen Zustand der Muskeln verbundenen Krankheiten, der Epilepsie und von akuten oder chronischen neurodegenerativen Krankheiten, aufweisende neue 2,3-Benzodiazepinderivate, ein Verfahren und Zwischenprodukte zu deren Herstellung und diese Verbindungen enthaltende pharmazeutische Präparate sowie ihre Verwendung zu schaffen.

Das Obige wurde überraschenderweise durch die Erfindung erreicht.

Es wurde nämlich überraschenderweise festgestellt, daß die oben geschilderte therapeutisch sehr wertvolle nicht-kompetitive AMPA-Rezeptor-antagonistische Wirkung bemerkenswerterweise auch dann auftritt, wenn im Benzolring der 2,3-Benzodiazepinderivate die Methylendioxygruppe durch 1 oder 2 Chlor- und/oder Bromatom(e) ersetzt wird, wobei diese neuen Verbindungen gegenüber den bekannten Derivaten vorteilhaftere Eigenschaften zeigen.

Die obige Feststellung ist umsomehr überraschend als bisher zur Ausübung der obigen Wirkung die Gegenwart der Methylendioxygruppe als unentbehrlich angesehen wurde.

Gegenstand der Erfindung sind daher neue 7- und/oder 8-halogensubstituierte 2,3-Benzodiazepinderivate der allgemeinen Formel

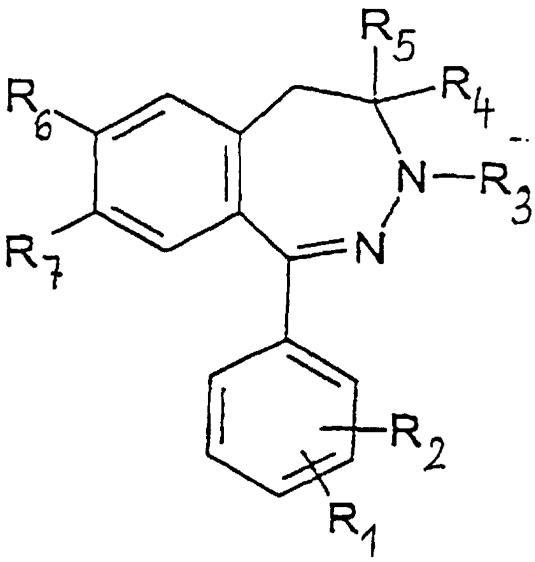

I,

worin

$R_1$ und $R_2$ unabhängig voneinander für Wasserstoff- beziehungsweise Halogenatome beziehungsweise $C_{1-4}$-Alkyl-, $C_{1-4}$-Alkoxy-, Nitro- beziehungsweise Trifluormethylgruppen beziehungsweise einen Aminorest der allgemeinen Formel

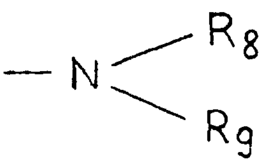

II

stehen,
in welchletzterer

$R_8$ und $R_9$ unabhängig voneinander Wasserstoffatome, $C_{1-4}$-Alkylreste bzw. Reste der allgemeinen Formel

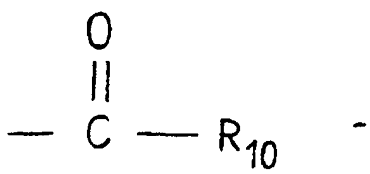

III

bedeuten,
in welchletzterer

$R_{10}$ ein Wasserstoffatom bzw. einen, gegebenenfalls durch 1 oder mehr $C_{1-4}$-Alkoxyrest(e) und/oder Halogenatom(e) substituierten, $C_{1-6}$-Alkyl-, $C_{6-10}$-Aryl-, $C_{1-4}$-Alkoxy-, $C_{3-5}$-Cycloalkyl-, $C_{2-6}$-Alkenyl- bzw. $C_{3-5}$-Cycloalkoxyrest bzw. Aminorest der allgemeinen Formel

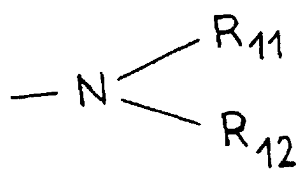

IV

darstellt, in welchletzterer

$R_{11}$ und $R_{12}$ unabhängig von-einander Wasserstoffatome bzw. $C_{1-4}$-Alkyl-, $C_{3-5}$-Cycloalkyl-bzw. $C_{6-10}$-Aryl-reste sind,

$R_3$ einen $C_{1-4}$-Alkyl- oder $C_{3-5}$-Cycloalkylrest bzw. einen Rest der allgemeinen Formel

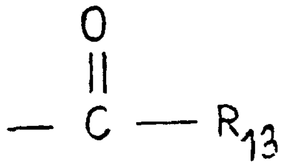

V

bedeutet,

in welchletzterer

$R_{13}$ dieselben Bedeutungen wie $R_{10}$ hat,

$R_4$ und $R_5$ unabhängig voneinander Wasserstoff bzw. $C_{1-3}$-Alkyl-reste darstellen und

$R_6$ und $R_7$ unabhängig voneinander für Wasserstoff-, Chlor-bzw. Bromatome stehen,

mit der weiteren Maßgabe, daß

mindestens 1 von

$R_6$ und $R_7$ von Wasserstoff verschieden ist,

sowie deren Isomere und die Säureadditionssalze dieser Verbindungen.

Die Alkyl- und Alkylenreste können geradkettig oder verzweigt sein. Die Cycloalkylreste können Cyclopropyl-, Cyclobutyl- und Cyclopentylreste sein.

Es ist bevorzugt, daß der/die Alkylrest(e), für welche[n] $R_1$ , $R_2$ , $R_8$ , $R_9$ , $R_{11}$ , $R_{12}$ und/oder $R_3$ stehen kann/ /können, [ein] solche[r] mit 1 bis 3, insbesondere 1 oder 2, ganz besonders 1, Kohlenstoffatom(en) ist/sind, der/die Alkoxyrest(e), welche[n] $R_1$ , $R_2$ , $R_{10}$ und/oder $R_{13}$ bedeuten kann/können und/oder durch welche[n] die $C_{1-6}$-Alkyl-, $C_{6-10}$-Aryl-, $C_{1-4}$-Alkoxy-, $C_{3-5}$-Cycloalkyl-, $C_{2-6}$-Alkenyl- bzw. $C_{3-5}$-Cycloalkoxyreste, für die $R_{10}$ und/oder $R_{13}$ stehen kann/können, substituiert sein kann/können, [ein] solche[r] mit 1 bis 3, insbesondere 1 oder 2, ganz besonders 1, Kohlenstoffatom(en) ist/sind, der/die Alkylrest(e), für welchen $R_{10}$ und/oder $R_{13}$ stehen kann/können, [ein] solche[r] mit 1 bis 4, insbesondere 1 bis 3, ganz besonders 1 oder 2, vor allem 1, Kohlenstoffatom(en) ist/sind, der/die Alkylrest (e), für welche[n] $R_4$ und/oder $R_5$ stehen kann/können, [ein] solche[r] mit 1 oder 2, insbesondere 1, Kohlenstoffatom (en) ist/sind, der/die Alkenylrest(e), für welche[n] $R_{10}$ und/oder $R_{13}$ stehen kann/können, [ein] solche[r] mit 2 bis 4, insbesondere 2 oder 3, ganz besonders 2, Kohlenstoffatomen, vor allem der/die Allylrest(e), ist/sind, der/die Cycloalkyl- oder Cycloalkoxyrest(e), welche[n] $R_{10}$ und/oder $R_{13}$ bedeuten kann/können und/ /oder der/die Cycloalkylrest(e), welche[n] $R_3$ , $R_{11}$ und/oder $R_{12}$ darstellen kann/können, [ein] solche[r] mit 3 oder 4, insbesondere 3, Kohlenstoffatomen ist/sind, der/die Arylrest(e), für welche[n] $R_{10}$ , $R_{13}$ , $R_{11}$ und/oder $R_{12}$ stehen kann/können, [ein] Phenyl- und/oder Naphthylrest(e), insbesondere der/die erstere(n), ist/sind und/oder das/die Halogenatom(e), für welche[s] $R_1$ und/oder $R_2$ stehen kann/können und/oder durch welche[s] die $C_{1-6}$-Alkyl-, $C_{6-10}$-Aryl-, $C_{1-4}$-Alkoxy-, $C_{3-5}$-Cycloalkyl-, $C_{2-6}$-Alkenyl- bzw. $C_{3-5}$-Cycloalkoxyreste, für die $R_{10}$ und/oder $R_{13}$ stehen kann/können, substituiert sein kann/können, [ein] Chlor- und/oder Bromatom(e), insbesondere das/die erstere(n), ist/sind.

Eine vorteilhafte Gruppe der erfindungsgemäßen 2,3-Benzodiazepinderivate sind diejenigen, bei welchen

$R_1$ für eine Aminogruppe in der 4-Stellung des Phenylrestes steht,

| | |
|---|---|
| $R_2$ , $R_4$ und $R_6$ | Wasserstoff bedeuten, |
| $R_5$ | den Methylrest darstellt, |
| $R_7$ | für ein Halogenatom steht und |
| $R_3$ | einen aliphatischen Acylrest oder Alkylcarbamoylrest bedeutet, |

sowie ihre Säureadditionssalze.

In einer besonders vorteilhaften Untergruppe steht $R_3$ für einen Acetyl-, Propionyl-, Cyclopropylcarbonyl- oder Methylcarbamoylrest.

Die Säureadditionssalze der erfindungsgemäßen 2,3-Benzodiazepinderivate der allgemeinen Formel I sind zweckmäßig solche mit pharmazeutisch brauchbaren Säuren. Sie können solche mit anorganischen Säuren, zum Beispiel Salzsäure, Bromwasserstoffsäure, Phosphorsäure oder Schwefelsäure, oder organischen Säuren, zum Beispiel Ameisensäure, Essigsäure, Maleinsäure, Fumarsäure, Bernsteinsäure, Milchsäure, Weinsäure, Zitronensäure oder Methansulfonsäure, sein.

Besonders bevorzugte erfindungsgemäße 2,3-Benzodiazepinderivate sind 3-[Acetyl]-1-[4'-(amino)-phenyl]-8-[chlor]-4-[methyl]-4,5-(di-[hydro]-3H-2,3-benzodiazepin und 1-[4'-(Amino)-phenyl]-8-[chlor]-4-[methyl]-3-[methylcarbamoyl]-4,5-di-[hydro]-3H-2,3-benzodiazepin sowie ihre Säureadditionssalze.

Die erfindungsgemäßen 2,3-Benzodiazepinderivate weisen mindestens 1 chirales Zentrum auf. Unter dem Ausdruck "Isomere" sind wegen des chiralen Zentrums die beiden Enantiomere und im Falle von bestimmten Substitutionen auf Grund der gegebenenfalls vorliegenden isomeren Verhältnisse die E- und Z-Isomere, Diastereomere, Tautomere oder deren Gemische, zum Beispiel Racemate, zu verstehen.

Gegenstand der Erfindung ist auch ein Verfahren zur Herstellung der erfindungsgemäßen 2,3-Benzodiazepinderivate der allgemeinen Formel I und ihrer Säureadditionssalze, welches dadurch gekennzeichnet ist, daß in an sich bekannter Weise

a) in 7- und/oder 8-halogensubstituierte 2,3-Benzodiazepinderivate, die in der 3-Stellung nicht substituiert sind, der allgemeinen Formel

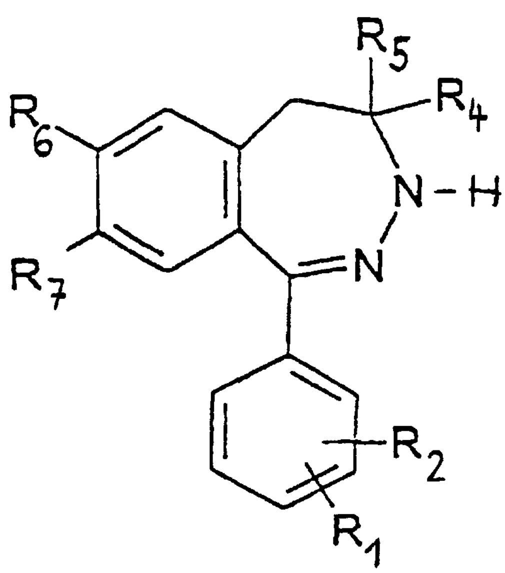

VI,

worin $R_1$ , $R_2$ , $R_4$ , $R_5$ , $R_6$ und $R_7$ wie oben festgelegt sind, bzw. ihre Säureadditionssalze in die 3-Stellung der gewünschte Substituent $R_3$ eingeführt wird oder

b) $N_2$-substituierte [<2'-(2''-{hydroxy}-alkyl)-4'- und/oder 5'-(halogen)-phenyl>-<gegebeneafalls substituierte phenyl>-methyliden]-hydrazinderivate der allgemeinen Formel

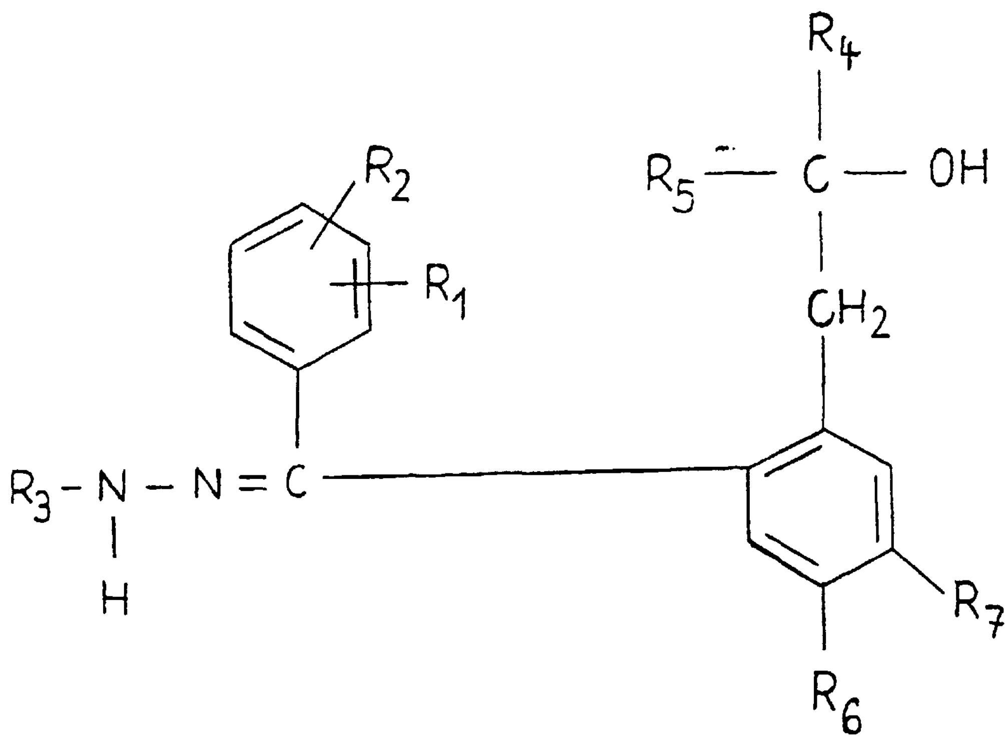

VII,

worin $R_1$, $R_2$, $R_3$, $R_4$, $R_5$, $R_6$ und $R_7$ wie oben festgelegt sind, mesyliert und unter alkalischen Bedingungen cyclisiert werden,
worauf in an sich bekannter Weise gegebenenfalls die erhaltenen 2,3-Benzodiazepinderivate der allgemeinen Formel I in andere der allgemeinen Formel I überführt werden und/oder die erhaltenen 2,3-Benzodiazepinderivate der allgemeinen Formel I in Säureadditionssalze überführt werden oder gegebenenfalls die erhaltenen Säureadditionssalze der 2,3-Benzodiazepinderivate der allgemeinen Formel I in die entsprechenden freien Basen der allgemeinen Formel I oder in andere Salze überführt werden und/oder gegebenenfalls eine Trennung der erhaltenen Isomergemische der 2,3-Benzodiazepinderivate der allgemeinen Formel I beziehungsweise von deren Säureadditionssalzen in die einzelnen Isomere beziehungsweise ein Überführen einzelner Isomere in Isomergemische vorgenommen wird.

Das erfindungsgemäße Verfahren kann, wie bereits gesagt, in an sich bekannter Weise durchgeführt werden (siehe zum Beispiel HU-PS 206 719 und US-PS 5 459 137).

Nach einer vorteilhaften Ausführungsform der Variante a) des erfindungsgemäßen Verfahrens werden die bei der 2,3-Benzodiazepin-Synthese üblichen Verfahrensweisen angewendet. Das Alkylieren der Aminogruppen kann mit Alkylhalogeniden oder durch mit Oxoverbindungen vorgenommenes reduktives Aminieren durchgeführt werden. Zum Acylieren werden vorzugsweise Säurechloride, Säureanhydride, gemischte Anhydride oder Chlorameisensäureester, wie Chlorameisensäurealkylester oder -phenylester, eingesetzt. Das Acylieren kann mit säurebindenden Mitteln und/oder Pyridinderivaten katalysiert werden. Die Umsetzung kann bei Raumtemperatur oder unter Erwärmen in einem Lösungsmittel durchgeführt werden.

Die Carbamoylgruppen können durch Umsetzen der 2,3-Benzodiazepinderivate der allgemeinen Formel II mit den entsprechenden Isocyanaten eingeführt werden. Es kann auch so verfahren werden, daß die 2,3-Benzodiazepinderivate der allgemeinen Formel II zuerst mit einem aktiven Chlorameisensäureester, zum Beispiel Chlorameisensäurephenylester, acyliert werden, worauf die erhaltenen Verbindungen mit primären oder sekundären Aminoverbindungen umgesetzt werden.

Ein Beispiel für das Überführen der so erhaltenen 2,3-Benzodiazepinderivate der allgemeinen Formel I in andere Verbindungen ist das katalytische Hydrieren einer Nitrogruppe zu einer Aminogruppe. Als Katalysator kann Raney-Nickel, Palladium oder Platin dienen. Als Wasserstoffquelle kann neben Wasserstoffgas auch Hydrazinhydrat oder Ammoniumformiat verwendet werden.

Die Salzbildung und Isomerentrennung können in an sich bekannter Weise durchgeführt werden.

Die bei der Variante a) des erfindungsgemäßen Verfahrens als Ausgangsstoffe verwendeten 2,3-Benzodiazepinderivate der allgemeinen Formel II, die in der 3-Stellung nicht substituiert sind, sind ebenfalls neu und gehören auch dem Gegenstand der vorliegenden Erfindung an. Die Herstellung der 2,3-Benzodiazepinderivate der allgemeinen

Formel II kann in an sich bekannter Weise, zum Beispiel wie in der HU-PS 191 702 beschrieben oder in Analogie zur Herstellung von bekannten Verbindungen, erfolgen.

Die bei der Variante b) des erfindungsgemäßen Verfahrens als Ausgangsstoffe verwendeten $N_2$-substituierten [<2'-(2''-{hydroxy}-alkyl)-4'- und/oder 5'-(halogen)-phenyl>-<gegebenenfalls substituierten phenyl>-methyliden]-hydrazinderivate können aus entsprechend substituierten Isochromanderivaten der allgemeinen Formel

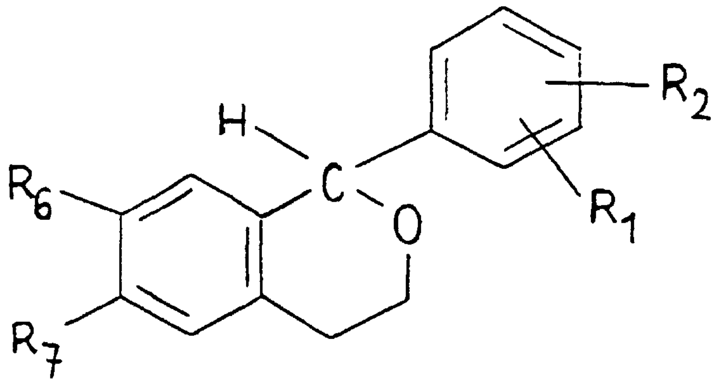

VIII,

worin $R_1$, $R_2$, $R_6$ und $R_7$ wie oben festgelegt sind, durch Oxydation mit Luft, vorteilhaft in Gegenwart von Basen, wie Alkalihydroxyden, und/oder der passenden Alkohole, zu den entsprechenden 1-(Hydroxy)-isochromanderivaten (Hemiketalen) der allgemeinen Formel

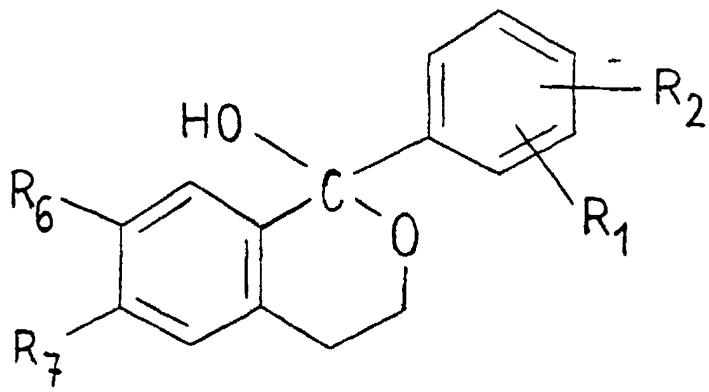

IX,

worin $R_1$, $R_2$, $R_6$ und $R_7$, wie oben festgelegt sind, und Einführen des substituierten Hydrazinrestes mit dem passenden Reagens, zum Beispiel Säurehydrazid oder Semicarbazid, vorteilhaft in Gegenwart des passenden Alkoholes, hergestellt werden. Anders ausgedrückt können nach der Variante b) des erfindungsgemäßen Verfahrens aus entsprechend substituierten Isochromanderivaten durch Oxydation mit Luft, vorteilhaft in Gegenwart von Basen, wie Alkalihydroxyden, und/oder der passenden Alkohole zu Hemiketalen und Einführen des Substituenten, wie einer Acylgruppe, für die herzustellenden, substituierten 2,3-Benzodiazepinderivate in deren 3-Stellung durch die passenden Reagenzien, wie Säurehydrazide oder Semicarbazide, vorteilhaft in Gegenwart der passenden Alkohole, erhaltene Ausgangsstoffe durch Mesylieren und alkalisches Cyclisieren zu den gewünschten substituierten 2,3-Benzodiazepinderivaten der allgemeinen Formel I umgesetzt werden.

Besonders vorteilhaft ist die Variante b) des erfindungsgemäßen Verfahrens zur Herstellung derjenigen substituierten 2,3-Benzodiazepinderivate, bei welchen $R_4$ für Wasserstoff steht und $R_5$ einen Alkylrest, insbesondere Methylrest, bedeutet, anwendbar. Ferner ist sie besonders vorteilhaft bei denjenigen substituierten 2,3-Benzodiazepinderivaten anwendbar, bei welchen $R_3$ für einen Rest der allgemeinen Formel V, insbesondere einen Acetylrest oder Methylcarbamoylrest, steht.

Wie bereits erwähnt, haben die erfindungsgemäßen 2,3-Benzodiazepinderivate der allgemeinen Formel I und ihre Säureadditionssalze eine bedeutende nicht-kompetitive AMPA-antagonistische Wirkung. Diese Verbindungen können in der Therapie als Spasmolytica, Muskelrelaxantien, zur Neuroprotektion und zur Behandlung von auf den erhöhten stimulierten Zustand des AMPA-Rezeptors zurückführbaren anderen neurologischen und psychiatrischen Störungen verwendet werden.

Gegenstand der Erfindung sind daher auch die erfindungsgemäßen 2,3-Benzodiazepinderivate der allgemeinen Formel I und ihre Säureadditionssalze zur Verwendung als Arzneimittel.

Ferner ist Gegenstand der Erfindung die Verwendung der erfindungsgemäßen 2,3-Benzodiazepinderivate der allgemeinen Formel I und ihrer Säureadditionssalze zur Herstellung von Arzneimitteln zur Behandlung von mit einem spastischen Zustand der Muskeln verbundenen Krankheiten, von Epilepsie und von akuten oder chronischen neurodegenerativen Krankheiten.

Die AMPA-antagonistische Wirkung von erfindungsgemäßen 2,3-Benzodiazepinderivaten der allgemeinen Formel I wurde mittels der nachstehenden Prüfversuche nachgewiesen.

Antagonisierung der Kainatwirkung (in vitro-Test)

Die Wirksamkeit in vitro der zu prüfenden Verbindungen wurde an einem isolierten Kückenretinapräparat bestimmt (M. J. Sheardown: Brain Res. 607 [1993], 189). Die "spreading depression" wurde mit 5μM Kainat hervorgerufen. Die $IC_{50}$-Werte wurden gemäß Sheardown bestimmt. Die Verbindungen wurden in mindestens 3 Konzentrationen geprüft. Die AMPA-antagonistischen Verbindungen hemmen das Ansprechen auf das AMPA-Rezeptor-agonistisch wirkende Kainat in einer konzentrationsabhängigen Weise. Die $IC_{50}$-Werte sind in der folgenden Tabelle zusammengestellt.

Tabelle

| Kainat-antagonistische Wirkung in vitro von 2,3-Benzodiazepinderivaten der allgemeinen Formel I | | |
|---|---|---|
| Verbindung | Beispiel Nr. | in vitro retinale "spreading depression" $IC_{50}$-Wert in μM |
| Bezeichnung | | |
| 3-[Acetyl]-1-[4'-(amino)-phenyl]-7-[chlor]-4-[methyl]-4,5-di-[hydro]-3H-2,3-benzodiazepin | 2 | 3,2 |
| 1-[4-(Amino)-phenyl]-7-[chlor]-4-[methyl]-3-[methylcarbamoyl]-4,5-di-[hydro]-3H-2,3-benzodiazepin | 4 | 3,6 |
| 3-[Acetyl]-1-[4'-(amino)-phenyl]-8-[chlor]-4-[methyl]-4,5-di-[hydro]-3H-2,3-benzodiazepin | 6 | 8,2 |
| 1-[4'-(Amino)-phenyl]-8-[chlor]-4-[methyl]-3-[methylcarbamoyl]-4,5-di-[hydro]-3H-2,3-benzodiazepin | 8 | 4,6 |
| 3-[Acetyl]-1-[4'-(amino)-phenyl]-7,8-di-[chlor]-4-[methyl]-4,5-di-[hydro]-3H-2,3-benzodiazepin | 10 | 2,5 |
| 1-[4'-(Amino)-phenyl]-7,8-di-[chlor]-4-[methyl]-3-[methylcarbamoyl]-4,5-di-[hydro]-3H-2,3-benzodiazepin | 12 | 1,2 |
| 5-[4'-(Amino)-phenyl]-9H-1,3-dioxolo[4,5-H][2,3] benzodiazepin | Vergleichssubstanz A HU-PS 191 698, Beispiel 8 | 9,5 |

Aus der obigen Tabelle geht die Überlegenheit der untersuchten erfindungsgemäßen 2,3-Benzodiazepinderivate gegenüber der Vergleichssubstanz hervor.

Gegenstand der Erfindung sind auch pharmazeutische Präparate, welche durch einen Gehalt an 1 oder mehr

erfindungsgemäßen 2,3-Benzodiazepinderivaten der allgemeinen Formel I und/oder Säureadditionssalz(en) derselben als Wirkstoff(en), zusammen mit 1 oder mehr üblichen pharmazeutischen Trägerstoff(en), Verdünnungsmittel(n), Lösungsmittel(n), Füllstoff(en) und/oder sonstigen Hilfsstoff(en) gekennzeichnet sind.

Die erfindungsgemäßen pharmazeutischen Präparate können als Träger- bzw. Füllstoff(e) [einen] organische[n] und/oder anorganische[n], zum Beispiel Wasser, Gelatine, Gummi arabicum, Milchzucker, Stärke, Magnesiumstearat, Talk, 1 oder mehr Pflanzenöl(e) und/oder Polyalkylenglykol(e) enthalten.

Die erfindungsgemäßen pharmazeutischen Präparate können fest, zum Beispiel Tabletten, Dragées, Suppositorien oder Kapseln, oder flüssig, zum Beispiel Lösungen, Suspensionen oder Emulsionen, sein. Sie können als weitere[n] Hilfsstoff(e) neben den oben erwähnten Komponenten zum Beispiel 1 oder mehr Konservierungsmittel, Stabilisiermittel, Emulgiermittel und/oder Puffer enthalten.

Die erfindungsgemäßen pharmazeutischen Präparate können zur enteralen oder parenteralen Verabreichung hergerichtet sein. Zur parenteralen Verabreichung eignen sich sterile Lösungen oder Suspensionen. Solche flüssigen Präparate können als weiteres Adjuvans Lokalanästhetica, Stabilisiermittel bzw. Puffer enthalten.

Die Wirkstoffdosis hängt von der Art der Verabreichung, dem Typ und der Schwere der Krankheit bzw. dem Gewicht und Alter des Patienten ab. Die tägliche Dosis beträgt im allgemeinen etwa 0,5 bis 1 000 mg, vorzugsweise 20 bis 200 mg, welche Dosis in 1 oder mehreren Portion(en) verabreicht werden kann.

Die im erfindungsgemäßen Verfahren als Ausgangssubstanzen eingesetzten 7- und/oder 8-halogensubstituierten 2,3-Benzodiazepinderivate der allgemeinen Formel II, die in der 3-Stellung nicht substituiert sind, einschließlich ihrer Isomere und Säureadditionssalze sind neu. Sie sind die Ursache für die überlegenen pharmakologischen Eigenschaften der erfindungsgemäßen 7- und/oder 8-halogensubstituierten 2,3-Benzodiazepinderivate der allgemeinen Formel I und ihrer Säureadditionssalze.

Gegenstand der Erfindung sind daher auch 7- und/oder 8-halogensubstituierte 2,3-Benzodiazepinderivate, die in der 3-Stellung nicht substituiert sind, der allgemeinen Formel

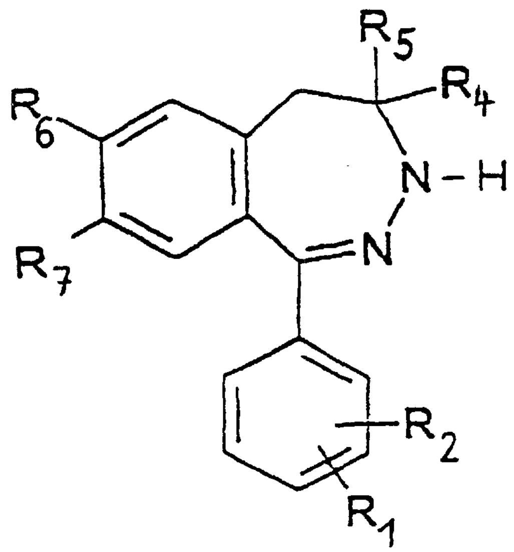

VI,

worin

$R_1$ , $R_2$ , $R_4$ , $R_5$ , $R_6$ und $R_7$ wie oben festgelegt sind,

sowie deren Isomere und die Säureadditionssalze dieser Verbindungen.

Die Erfindung wird an Hand der folgenden Beispiele näher erläutert, wobei die Verhältnisse der für die Chromatographie verwendeten Lösungsmittel Volumverhältnisse sind.

<u>Beispiel 1</u>

3-[Acetyl]-7-[chlor]-4-[methyl]-1-[4'-(nitro)-phenyl]-4,5-di-[hydro]-3H-2,3-benzodiazepin

0,72 g (2, 2 Millimole) 7-[Chlor]-4-[methyl]-1-[4'-(nitro)-phenyl]-4,5-di-[hydro]-3H-2,3-benzodiazepin werden in 4 ml Essigsäureanhydrid 3 Stunden lang bei 25°C gerührt. Das Reaktionsgemisch wird in 20 ml eiskaltes Wasser

gegossen, das kristalline Produkt wird abfiltriert und mit Wasser mehrmals gewaschen. Das so erhaltene Rohprodukt wird in 4 ml heißem Äthanol suspendiert, abfiltriert und getrocknet. Es werden 0,69 g der im Titel genannten Verbindung erhalten, Ausbeute 88%, F.: 174-175°C.

Der Ausgangsstoff wird wie folgt hergestellt:

A.) 6-[Chlor]-3-[methyl]-1-[4'-(nitro)-phenyl]-isochroman

Einer Lösung von 17,06 g (100 Millimole) 1-[3'-(Chlor)-phenyl]-2-propanol [J. Med. Chem. 21, 454 (1978)] und 15,11 g (100 Millimole) 4-(Nitro)-benzaldehyd in 100 ml wasserfreiem Benzol werden 13,65 g (100 Millimole) frisch geglühtes Zinkchlorid zugegeben, worauf in das Reaktionsgemisch 3 Stunden lang wasserfreies Chlorwasserstoffgas eingeleitet wird. Das Reaktionsgemisch wird 2,5 Stunden lang zum Sieden erhitzt, dann abgekühlt und mit 100 ml Wasser gerührt. Die Phasen werden voneinander abgetrennt. Die organische Phase wird zuerst mit Wasser und einer Natriumbicarbonatlösung, danach mit einer gesättigten Natriumchloridlösung gewaschen, getrocknet und eingeengt. Das ölige Rohprodukt (30,95 g) wird aus 200 ml heißem Äthanol kristallisiert. Es werden 23,49 g der im Titel genannten Verbindung erhalten, Ausbeute 66%, F.: 118-120°C.

B.) 6-[Chlor]-3-[methyl]-1-[4'-(nitro)-phenyl]-2-benzopyrilium-perchlorat

26,9 g (88,5 Millimole) des nach Stufe A.) hergestellten Isochromanderivates werden in 270 ml Aceton gelöst, worauf der Lösung unter Eiskühlung innerhalb einer Stunde 116 ml (310 Millimole) Jones-Reagens tropfenweise zugegeben werden. Das Reaktionsgemisch wird bei 25°C 4 Stunden lang gerührt. Das ausgeschiedene Chromsalz wird filtriert und das Filtrat eingeengt.

Der kristalline Rückstand wird in 100 ml Wasser suspendiert und abfiltriert. Die erhaltene kristalline Substanz wird in 100 ml Wasser suspendiert und wieder abfiltriert. Die erhaltene kristalline Substanz wird in 340 ml heißem Eisessig gelöst. Nach Zugabe von 5,91 ml 70 gew.-%-iger Perchlorsäure wird das ausgeschiedene kristalline Produkt filtriert und viermal mit je 100 ml Eisessig gewaschen. Es werden 6,84 g der im Titel genannten Verbindung erhalten, Ausbeute 19%, F. : 236-237°C (Zersetzung).

C.) 7-[Chlor]-4-[methyl]-1-[4'-(nitro)-phenyl]-5H-2,3-benzodiazepin

Einer Lösung von 6,43 g (16 Millimole) des nach Stufe B.) hergestellten Benzopyrilium-perchlorats in 32 ml Dimethylformamid werden 2,31 ml (48 Millimole) 98 gew.-%-iges Hydrazinhydrat tropfenweise zugegeben. Das Reaktionsgemisch wird eine Stunde lang bei 25°C gerührt, danach in 320 ml Wasser gegossen. Das ausgeschiedene Produkt wird abfiltriert, fünfmal mit je 10 ml Wasser gewaschen. Das Rohprodukt wird in 50 ml heißem Äthanol suspendiert. Es werden 4,41 g der im Titel genannten Verbindung erhalten, Ausbeute 87%, F.: 227-228°C.

D.) 7-[Chlor]-4-[methyl]-1-[4'-(nitro)-phenyl]-4,5-di-[hydro]-3H-2,3-benzodiazepin

1,5 g (4,78 Millimole) des nach Stufe C.) hergestellten Benzodiazepinderivates werden in 60 ml Methanol suspendiert, worauf der Suspension zuerst 4,64 ml (57,3 Millimole) konzentrierter Salzsäure und danach in Portionen unter Wasserkühlung 2,07 g (54,8 Millimole) Natriumborhydrid zugegeben werden. Die entstandene Suspension wird bei 25°C eine Stunde lang gerührt, der pH-Wert wird durch Zugabe von festem Natriumcarbonat auf 8 eingestellt. Das Gemisch wird mit 60 ml Wasser verdünnt. Das ausgeschiedene Produkt wird viermal mit je 5 ml 50 gew.-%-igem wäßrigem Methanol gewaschen und getrocknet. Es werden 1,47 g der im Titel genannten Verbindung erhalten, Ausbeute 97,4%, F.: 152-154°C.

Beispiel 2

3-[Acetyl]-1-[4'-(amino)-phenyl]-7-[chlor]-4-[methyl]-4,5-di-[hydro]-3H-2,3-benzodiazepin

0,66 g (1,8 Millimole) 3-[Acetyl]-7-[chlor]-4-[methyl]-1-[4'-(nitro)-phenyl]-4,5-di-[hydro]-3H-2,3-benzodiazepin (Beispiel 1) werden in 35 ml Methanol suspendiert, worauf etwa 0,5 g feuchter Raney-Nickel-Katalysator und unter starkem Rühren 0,32 ml (6,5 Millimole) 98 gew.-%-iges Hydrazinhydrat zugegeben werden. Das Reaktionsgemisch wird weitere 45 Minuten lang gerührt, worauf der Katalysator abfiltriert, mit Methanol gewaschen und das Filtrat eingeengt wird. Der Rückstand wird mit 10 ml Wasser behandelt. Das feste Rohprodukt (0,54 g) wird aus 2 ml Äthanol umkristallisiert. Es werden 0,44 g der im Titel genannten Verbindung erhalten, Ausbeute 75%, F.: 90-92°C.

Beispiel 3

7-[Chlor]-4-[methyl]-3-[methylcarbamoyl]-1-[4'-(nitro)-phenyl]-4,5-di-[hydro]-3H-2,3-benzodiazepin

Einer Lösung von 0,72 g (2, 2 Millimole) 7-[Chlor]-4-[methyl]-1-[4'-(nitro)-phenyl]-4,5-di-[hydro]-3H-2,3-benzodiazepin (hergestellt nach Beispiel 1 D.) in 15 ml wasserfreiem Dichlormethan werden 1,3 ml (22,10 Millimole) Methylisocyanat zugefügt. Das Reaktionsgemisch wird bei 25°C 10 Tage lang stehen gelassen. Das Gemisch wird eingeengt und der Rückstand aus 3 ml heißem Äthanol umkristallisiert. Das Produkt wird abfiltriert, dreimal mit je 1 ml Äthanol gewaschen und getrocknet. Es werden 0,78 g der im Titel genannten Verbindung erhalten, Ausbeute 95%, F.: 224-226°C.

Beispiel 4

1-[4-(Amino)-phenyl)-7-[chlor]-4-[methyl]-3-[methylcarbamoyl]--4,5-di-[hydro]-3H-2,3-benzodiazepin

0,75 g (2,0 Millimole) des nach Beispiel 3 hergestellten 7-[Chlor]-4-[methyl]-3-[methylcarbamoyl]-1-[4'-(nitro)-phenyl]-4,5-di-[hydro]-3H-2,3-benzodiazepins werden in analoger Weise wie im Beispiel 2 reduziert. Das erhaltene Rohprodukt wird aus heißem Äthanol umkristallisiert. Es werden 0,55 g der im Titel genannten Verbindung erhalten, Ausbeute 80%, F.: 134-136°C.

Beispiel 5

3-[Acetyl]-8-[chlor]-4-[methyl]-1-[4'-(nitro)-phenyl]-4,5-di-[hydro]-3H-2,3-benzodiazepin

0,69 g (2,19 Millimole) 8-[Chlor]-4-[methyl]-1-[4'-(nitro)-phenyl]-4,5-di-[hydro]-3H-2,3-benzodiazepin werden in analoger Weise wie im Beispiel 1 acetyliert. Es werden 0,70 g der im Titel genannten Verbindung erhalten, Ausbeute 89%, F.: 227-229°C.

Der Ausgangsstoff wird wie folgt hergestellt:

A.) 7-[Chlor]-3-[methyl]-1-[4'-(nitro)-phenyl]-isochroman

Man verfährt wie im Beispiel 1 A.) mit dem Unterschied, daß man als Ausgangsstoff 11,94 g (70 Millimole) 1-[4'-(Chlor)-phenyl]-2-propanol [J. Med. Chem. 21, 454 (1978)] verwendet und das Reaktionsgemisch 1,5 Stunden lang zum Sieden erhitzt. Es werden 6,8 g der im Titel genannten Verbindung erhalten, Ausbeute 32%, F.: 120-123°C.

B.) 7-[Chlor]-3-[methyl]-1-[4'-(nitro)-phenyl]-2-benzopyrilium-perchlorat

6,8 g (22,4 Millimole) des nach Stufe A.) hergestellten Isochromanderivates werden in analoger Weise wie im Beispiel 1 B.) mit Jones Reagens oxydiert. Das Produkt wird in Eisessig-Lösung durch Behandlung mit Perchlorsäure hergestellt. Es werden 3,73 g der im Titel genannten Verbindung erhalten, Ausbeute 42%, F.: 247-255°C.

C.) 8-[Chlor]-4-[methyl]-1-[4'-(nitro)-phenyl]-5H-2,3-benzodiazepin

4,1 g (10,25 Millimole) des nach Stufe B.) hergestellten Benzopyriliumperchlorats werden einer mit kaltem Wasser gekühlten Lösung von 20,5 ml Dimethylformamid und 1,5 ml (70,7 Millimole) 98 gew.-%-igem Hydrazinhydrat zugegeben. Das Reaktionsgemisch wird 1,5 Stunden lang bei 25°C gerührt. Nach Zugabe von 25 ml Wasser wird das ausgeschiedene Rohprodukt abfiltriert, viermal mit je 5 ml Wasser gewaschen und aus 25 ml Isopropanol umkristallisiert. Es werden 2,82 g der im Titel genannten Verbindung erhalten, Ausbeute 87%, F.: 199-203°C.

D.) 8-[Chlor]-4-[methyl]-1-[4'-(nitro)-phenyl]-4,5-di-[hydro]-3H-2,3-benzodiazepin

1,62 g (5,16 Millimole) des nach Stufe C.) hergestellten Benzodiazepinderivates werden in analoger Weise wie im Beispiel 1 D.) reduziert. Es werden 1,59 g der im Titel genannten Verbindung erhalten, Ausbeute 98%, F.: 132-135°C.

Beispiel 6

3-[Acetyl]-1-[4'-(amino)-phenyl]-8-[chlor]-4-[methyl]-4,5-di-[hydro]-3H-2,3-benzodiazepin

0,81 g (2,26 Millimole) des nach Beispiel 5 hergestellten 3-[Acetyl]-8-[chlor]-4-[methyl]-1-[4'-(nitro)-phenyl]-4,5-di-[hydro]-3H-2,3-benzodiazepins werden in analoger Weise wie im Beispiel 2 reduziert. Das Rohprodukt wird aus 50 gew.-%-igem wäßrigem Äthanol umkristallisiert. Es werden 0,64 g der im Titel genannten Verbindung erhalten, Ausbeute 86%, F.: 187-189°C.

Beispiel 7

8-[Chlor]-4-[methyl]-3-[methylcarbamoyl]-1-[4'-(nitro)-phenyl)-4,5-di-[hydro]-3H-2,3-benzodiazepin

0,79 g (2,5 Millimole) des nach Beispiel 5 hergestellten 8-[Chlor]-4-[methyl]-1-[4'-(nitro)-phenyl]-4,5-di-[hydro]-3H-2,3-benzodiazepins werden in analoger Weise wie im Beispiel 3 zwecks Einführung der Methylcarbamoylgruppe der Behandlung mit Methylisocyanat unterworfen. Das Rohprodukt wird aus Isopropanol umkristallisiert. Es werden 0,85 g der im Titel genannten Verbindung erhalten, Ausbeute 91%, F.: 190-192°C.

Beispiel 8

1-[4-'(Amino)-phenyl]-8-[chlor]-4-[methyl]-3-[methylcarbamoyl]-4,5-di-[hydro]-3H-2,3-benzodiazepin

0,65 g (1,74 Millimole) des nach Beispiel 7 hergestellten 8-[Chlor]-4-[methyl]-3-[methylcarbamoyl]-1-[4'-(nitro)-phenyl]-4,5-di-[hydro]-3H-2,3-benzodiazepins werden analog wie im Beispiel 2 reduziert. Es werden 0,59 g der im Titel genannten Verbindung erhalten, Ausbeute 99%, F.: 115-118°C.

Beispiel 9

3-[Acetyl]-7,8-di-[chlor]-4-[methyl]-1-[4'-(nitro)-phenyl]-4,5-di-[hydro]-3H-2,3-benzodiazepin

0,35 g (0,99 Millimole) 7,8-Di-[chlor]-4-[methyl]-1-[4'-(nitro)-phenyl]-4,5-di-[hydro]-3H-2,3-benzodiazepin werden analog wie im Beispiel 1 acetyliert. Es werden 0,36 g der im Titel genannten Verbindung erhalten, Ausbeute 93%, F.:198-200°C.

Der Ausgangsstoff wird wie folgt hergestellt:

A.) 6,7-Di-[chlor]-3-[methyl]-1-[4'-(nitro)-phenyl]-isochroman

Man verfährt wie im Beispiel 1 A.), mit dem Unterschied, daß man als Ausgangsstoff 10,47 g (51,0 Millimole) 1-[3',4'-Di-(chlor)-phenyl]-2-propanol verwendet und das Reaktionsgemisch 3 Stunden lang zum Sieden erhitzt. Es werden 4,29 g der im Titel genannten Verbindung erhalten, Ausbeute 25%, F.: 189-191°C.

B.) 7,8-Di-[chlor]-4-[methyl]-1-[4'-(nitro)-phenyl]-5H-2,3-benzodiazepin

4,75 g (14,0 Millimole) des nach Stufe A.) hergestellten Isochromanderivates werden analog wie im Beispiel 1 B.) mit Jones-Reagens oxydiert, mit dem Unterschied, daß anstatt der Perchlorsäurebehandlung des Rohproduktes das 1-Acetonyl-3,4-dichlor-4-nitro-benzophenon-Derivat isoliert (F.: 121-122°C) und dessen mit Isopropanol gebildete Lösung bei 25°C mit 98 gew.-%-igem Hydrazinhydrat umgesetzt wird. Das so erhaltene Monohydrazon-Derivat (F.: 167-169°C) wird mit 15 gew.-%-igem methanolischem Chlorwasserstoff umgesetzt, wonach aus dem Hydrochlorid der im Titel genannten cyclischen Verbindung die Base mit Triäthylamin freigesetzt wird. Die Base wird zwecks Reinigung aus heißem Dimethylformamid umkristallisiert. Die im Titel genannte Verbindung schmilzt bei 231-233°C.

C.) 7,8-Di-[chlor]-4-[methyl]-1-[4'-(nitro)-phenyl]-4,5-di-[hydro]-3H-2,3-benzodiazepin

0,74 g (2,1 Millimole) des nach Stufe B.) hergestellten Benzodiazepinderivates werden analog wie im Beispiel 1 D.) reduziert. Es werden 0,70 g der im Titel genannten Verbindung erhalten, Ausbeute 95%, F.: 182-184°C.

Beispiel 10

3-[Acetyl]-1-[4'-(amino)-phenyl]-7,8-di-[chlor]-4-[methyl]-4,5-di-[hydro]-3H-2,3-benzodiazepin

0,34 g (0,86 Millimole) des nach Beispiel 9 hergestellten 3-[Acetyl]-7,8-di-[chlor]-4-[methyl]-1-[4'-(nitro)-phenyl]-4,5-di-[hydro]-3H-2,3-benzodiazepins werden analog wie im Beispiel 2 reduziert. Das Rohprodukt wird durch Suspendieren in heißem Äthanol gereinigt. Es werden 0,25 g der im Titel genannten Verbindung erhalten, Ausbeute 80%, F.: 242-243°C.

Beispiel 11

7,8-Di-[chlor]-4-[methyl]-3-[methylcarbamoyl]-1-[4'-(nitro)-phenyl]-4,5-di-[hydro]-3H-2,3-benzodiazepin

Man verfährt wie im Beispiel 3, mit dem Unterschied, daß man als Ausgangsstoff 0,33 g (0,94 Millimole) des nach Beispiel 9 C.) hergestellten 7,8-Di-[chlor]-4-[methyl]-1-[4'-(nitro)-phenyl]-4,5-di-[hydro]-3H-2,3-benzodiazepins verwendet. Das Rohprodukt wird durch Suspendieren in heißem Äthanol gereinigt. Es werden 0,37 g der im Titel genannten Verbindung erhalten, Ausbeute 97%, F.: 221-223°C.

Beispiel 12

1-[4'-(Amino)-phenyl]-7,8-di-[chlor]-4-[methyl]-3-[methylcarbamoyl]-4,5-di-[hydro]-3H-2,3-benzodiazepin

0,35 g (0,85 Millimole) des nach Beispiel 11 hergestellten 7,8-Di-[chlor]-4-[methyl]-3-[methylcarbamoyl]-1-[4'-(nitro)-phenyl]-4,5-di-[hydro]-3H-2,3-benzodiazepins werden analog wie im Beispiel 2 reduziert. Das Rohprodukt wird aus 50 gew.-%-igem wäßrigem Äthanol umkristallisiert. Es werden 0,27 g der im Titel genannten Verbindung erhalten, Ausbeute 84%, F.: 224-225°C.

Beispiel 13

3-[Acetyl]-8-[chlor]-4-[methyl]-1-[4'-(nitro)-phenyl]-4,5-di-[hydro]-3H-2,3-benzodiazepin

(ein vom Beispiel 5 verschiedenes Verfahren)

6,35 g (etwa 16,3 Millimole) rohes Essigsäure-1-[<2'-(2''-{hydroxy}-propyl)-5'-(chlor)-phenyl>-<4'''-(nitro)-phenyl>-methyliden]-hydrazid (hergestellt nach Stufe B.) werden in 70 ml wasserfreiem Dichlormethan gelöst. Die Lösung wird auf -15°C gekühlt, worauf unter Erwärmen 4,10 ml (29,3 Millimole) Triäthylamin und danach innerhalb von 5 Minuten 1,65 ml (21,2 Millimole) Mesylchlorid zugetropft werden. Das Reaktionsgemisch wird 20 Minuten lang gerührt, wonach mit 30 ml 1 N Salzsäure und zweimal mit je 30 ml eiskalter Natriumchloridlösung gewaschen, getrocknet und eingeengt wird. Der erhaltene schaumartige Rückstand wird in 60 ml Äthanol aufgenommen und der Suspension wird unter Eiskühlung 0,90 ml (17,1 Millimole) einer 50 gew.-%-igen Natriumhydroxydlösung zugetropft. Der größte Teil des Zwischenproduktes geht in Lösung und nach einigen Minuten beginnt die Ausscheidung von gelben Kristallen. Das Gemisch wird bei Raumtemperatur weitere 4 Stunden lang gerührt. Dem Reaktionsgemisch werden unter Eiskühlung innerhalb von 45 Minuten 100 ml Wasser zugetropft. Der Niederschlag wird abfiltriert und mit Wasser gewaschen. Das erhaltene trockene Rohprodukt wird in etwa 450 ml heißem Äthanol gelöst und die Lösung wird auf etwa 1/3 des Volumens eingeengt. Nach Abkühlen und Filtrieren werden 3,92 g der im Titel genannten Verbindung erhalten, Ausbeute 67% (auf das im Beispiel 5 A.) beschriebene Isochromanderivat bezogen). Das gelbe kristalline Produkt schmilzt bei 226-228°C.

Der Ausgangsstoff wird wie folgt hergestellt:

A.) 1-[Hydroxy]-7-[chlor]-3-[methyl]-1-[4'-(nitro)-phenyl]-isochroman

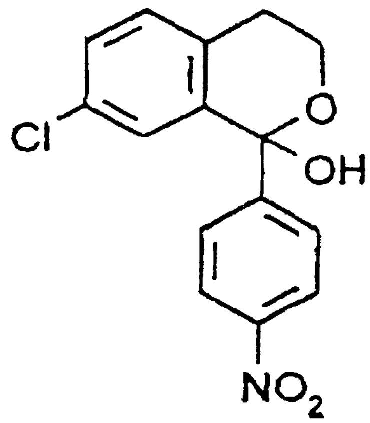

x.

7,44 g (24,5 Millimole) 7-[Chlor]-3-[methyl]-1-[4'-(nitro)-phenyl]-isochroman (Beispiel 5 A.) werden in einer Mischung von 50 ml Dimethylformamid und 24 ml Dimethylsulfoxid gelöst. Die Lösung wird mit eiskaltem Wasser gekühlt, wobei durch ein unter die Oberfläche der Flüssigkeit geleitetes Kapillarrohr Luft eingeführt wird. Unter hef tiger Blasenbildung werden der Lösung 2,60 ml (49,0 Millimole) einer 50 gew.-%-igen wäßrigen Natriumhydroxydlösung zugegeben, wobei das Gemisch eine violettschwarze Farbe annimmt. Das Reaktionsgemisch wird 2 Stunden lang gerührt, wonach es in 240 ml eiskalte 0,5 N Salzsäure gegossen wird. Der ausgeschiedene Niederschlag wird dreimal mit je 100 ml Äthylacetat extrahiert. Die organische Phase wird mit 100 ml einer gesättigten Natriumbicarbonatlösung und einer Kochsalzlösung gewaschen, getrocknet und eingeengt. Der schaumartige Rückstand (8,70 g) ist nach der Dünnschichtchromatographie einheitlich (n-Hexan : Äthylacetat = 4 : 1 : $R_f$ = etwa 0,2).
Nach $^{1}H$ NMR ist die Verbindung ein Gemisch der beiden möglichen Stereoisomere und die Reinheit beträgt etwa 90%. Das Produkt wird in der nächsten Stufe in dieser Form verwendet.

B.) Essigsäure-1-[<2'-(2''-{hydroxy}-propyl)-5'-(chlor)-phenyl>-<4'''-(nitro)-phenyl>-methyliden]-hydrazid

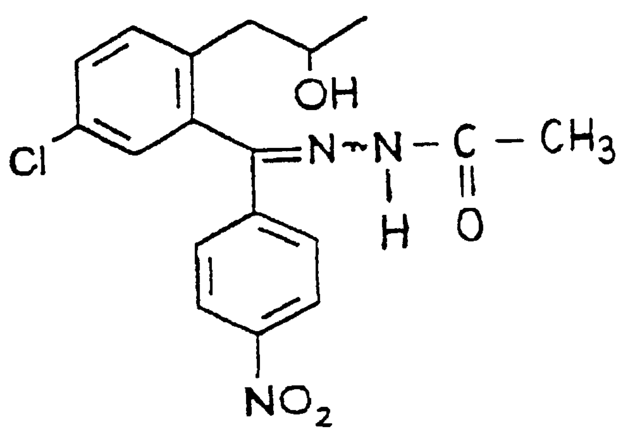

xI.

Ein Gemisch von 5,85 g (16,3) Millimole des etwa 89-90 gew.-%-igen Hemiketales 1-[Hydroxy]-7-[chlor]-3-[methyl]-1-[4'-(nitro)-phenyl]-isochroman (hergestellt nach Stufe A.), 1,45 g (19,5 Millimole) Essigsäurehydrazid, 80 ml Isopropanol und 20 ml Wasser wird nach Zugabe von 2ml 1 N Salzsäure zum Sieden erhitzt. Am Anfang der Reaktion gehen die Reagenzien in Lösung. Das Reaktionsgemisch wird 3,5 Stunden lang zum Sieden erhitzt, wonach eine weitere Menge von 0,33 g (4,45 Millimole) Essigsäurehydrazid und 1 ml 1 N Salzsäure zugegeben werden. Die Reaktion wird weitere 3,5 Stunden lang fortgesetzt und danach eingeengt. Der Rückstand wird in 150 ml Äthylacetat und 100

ml Natriumcarbonatlösung gelöst. Die Phasen werden voneinander getrennt, die wäßrige Schicht wird mit 50 ml Äthylacetat extrahiert. Die vereinigten organischen Phasen werden mit einer Kochsalzlösung gewaschen, getrocknet und eingeengt. Das Benzol wird über dem Rückstand zwecks Trocknen abgedampft. Das erhaltene gelbe Harz (6,35 g) wird bei der Mesylierungs- und Ringschlußreaktion verwendet.

Beispiel 14

3-[Carbamoyl]-8-[chlor]-4-[methyl]-1-[4'-(nitro)-phenyl]-4,5-di-[hydro]-3H-2,3-benzodiazepin

6,13 g (etwa 16,27 Millimole) rohes 1-[<2'-(2''-{hydroxy}-propyl)-5'-(chlor)-phenyl>-<4'''-(nitro)-phenyl>-methyliden]-semicarbazid werden in 60 ml wasserfreiem Pyridin gelöst, worauf der Lösung bei -5°C unter Rühren 1,76 ml (22,77 Millimole) Mesylchlorid zugetropft werden. Das Reaktionsgemisch wird 23 Stunden lang bei Raumtemperatur gerührt. Nach Zugabe von 0,17 ml (2,9 Millimole) Mesylchlorid wird das Gemisch 3 Stunden lang gerührt, in 500 ml 1,5 N eiskalte Salzsäure gegossen und das Produkt wird dreimal mit je 90 ml Dichlormethan extrahiert. Die organische Lösung wird mit einer Kochsalzlösung gewaschen, getrocknet und eingeengt. Das als gelber Schaum erhaltene Zwischenprodukt wird in 100 ml Äthanol aufgenommen und 1,03 ml (19,5 Millimole) einer 50 gew.-%-igen wäßrigen Natriumhydroxydlösung werden bei Raumtemperatur unter Rühren zugetropft. Das Gemisch wird 4 Stunden lang unter Eiskühlung gerührt, wonach unter Eiskühlung 150 ml Wasser zugetropft werden. Das ausgeschiedene kristalline Produkt wird abfiltriert und mit Wasser gewaschen. Es werden 4,18 g der rohen Titelverbindung erhalten, Ausbeute 76%. Das Rohprodukt wird aus 270 ml Äthanol durch Einengen auf etwa 1/3 seines Volumens umkristallisiert. Es werden 3,16 g der im Titel genannten Verbindung erhalten, Ausbeute 55%, F.: 233-234°C (Zersetzung).
Der Ausgangsstoff wird wie folgt hergestellt:

1-[<2'-(2''-{hydroxy}-propyl)-5'-(chlor)-phenyl>-<4'''-(nitro)-phenyl>-methyliden]-semicarbazid

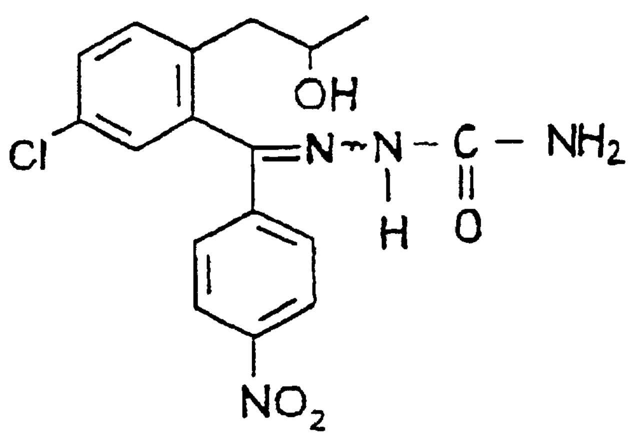

XII.

Ein Gemisch von 6,19 g (etwa 18 Millimole) des nach Beispiel 13 A.) hergestellten Hemiketals 1-[Hydroxy]-7-[chlor]-3-[methyl]-1-[4'-(nitro)-phenyl]-isochroman, 3,01 g (27 Millimole) Semicarbazid-hydrochlorid, 140 ml Isopropanol und 60 ml Wasser wird 5 Stunden lang zum Sieden erhitzt. Dem Reaktionsgemisch wird weiteres 0,60 g (5,4 Millimole) Semicarbazid-hydrochlorid zugesetzt, worauf das Gemisch weitere 5 Stunden lang zum Sieden erhitzt wird. Das Gemisch wird eingeengt, der Rückstand in Wasser suspendiert, abfiltriert und mit Wasser gewaschen. Das erhaltene Produkt (6,62 g, 98%) wird bei der Mesylierung und Ringschlußreaktion in dieser Form verwendet.
Eine Probe für analytische Zwecke wird durch Reinigung mit Hilfe von Säulenchromatographie (Silikagel; 95:5 Mischung von Chloroform und Methanol) hergestellt. Nach $^1$H NMR ist das Produkt ein etwa 1:1 Gemisch der Stereoisomere, die Reinheit beträgt etwa 90%. Für das im Massenspektrum erhaltene Molekülion M = 376/378.

Beispiel 15

1-[4'-(Amino)-phenyl]-3-[carbamoyl]-8-[chlor]-4-[methyl]-4,5-di-[hydro]-3H-2,3-benzodiazepin

3,16 g (8,81 Millimole) 3-[Carbamoyl]-8-[chlor]-4-[methyl]-1-[4'-(nitro)-phenyl]-4,5-di-[hydro]-3H-2,3-benzodiazepin (Beispiel 14) werden in Methanol in Gegenwart eines Raney-Nickel-Katalysators mit 5 Moläquivalenten Hydrazinhydrat in Analogie zum Beispiel 2 reduziert. Das Rohprodukt (2,71 g) wird aus 50 gew.-%-igem wäßrigem Ähtanol umkristallisiert. Es werden 2,29 g der im Titel genannten Verbindung erhalten, Ausbeute 79%, F.: 218-220°C.

Beispiel 16

3-[Äthoxycarbonyl]-8-[chlor]-4-[methyl]-1-[4'-(nitro)-phenyl]-4,5-di-[hydro]-3H-2,3-benzodiazepin

2,36 g (5,81 Millimole) 3-[<2'-(2''-{Hydroxy}-propyl)-5'-(chlor)-phenyl>-<4'-(nitro)-phenyl>-methyliden]-äthylcarbazat werden in 25 ml wasserfreiem Dichlormethan gelöst. Nach Zugabe von 1,46 ml (10,43 Millimole) Triäthylamin werden bei -15°C 0,60 ml (7,70 Millimole) Mesylchlorid zugegeben. Das Reaktionsgemisch wird eine Stunde lang gerührt, mit 20 ml Dichlormethan verdünnt und nacheinander mit eiskalter 1 N Salzsäure und einer Kochsalzlösung gewaschen. Nach Trocknen und Einengen wird das als Rückstand verbliebene Harz in 34 ml Äthanol gelöst. Unter Rühren werden 0,32 ml (6,1 Millimole) einer 50 gew.-%-igen wäßrigen Natriumhydroxydlösung zugegeben. Das Reaktionsgemisch wird 2 Stunden lang gerührt, wonach unter Eiskühlung 100 ml Wasser zugetropft werden. Das ausgeschiedene Produkt wird abfiltriert und mit Wasser gewaschen. Es werden 1,83 g der im Titel genannten Verbindung erhalten, Ausbeute 81%, F.: 124-126°C.

Der Ausgangsstoff wird wie folgt hergestellt:

3-[<2'-(2''-{Hydroxy}-propyl)-5'-(chlor)-phenyl>-<4'-(nitro)-phenyl>-methyliden]-äthylcarbazat

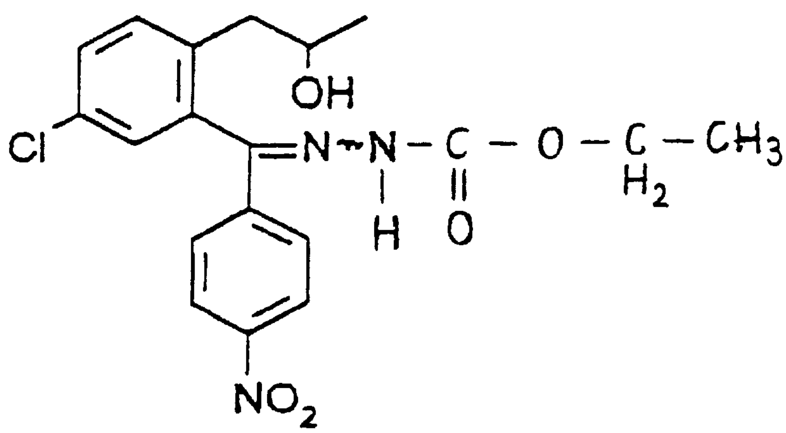

XIII.

Ein Gemisch von 2,10 (etwa 6 Millimole) des nach Beispiel 13 A.) hergestellten Hemiketals 1-[Hydroxy]-7-[chlor]-3-[methyl]-1-[4'-(nitro)-phenyl]-isochroman, 1,25 g (12, 0 Millimole) Äthylcarbazat, 80 ml Äthanol oder Isopropanol und 60 ml Wasser wird unter Zugabe von 0,1 ml konzentrierter Salzsäure zum Sieden erhitzt. Nach 2 Stunden werden weitere 0,12 g (1,2 Millimole) Äthylcarbazat und ein Tropfen Salzsäure zugegeben und das Gemisch wird weitere 2 Stunden lang zum Sieden erhitzt. Nach Einengen wird der Rückstand in eine Natriumcarbonatlösung aufgenommen. Das kristalline Produkt wird abfiltriert und mit Wasser gewaschen. Es werden 2,36 g der im Titel genannten Verbindung in Form eines etwa 1:1 Isomerengemisches erhalten, Ausbeute 97%, F.: 123-125°C.

Beispiel 17

1-[4'-(Amino)-phenyl]-3-[äthoxycarbonyl]-8-[chlor]-4-[methyl]-4,5-di-[hydro]-3H-2,3-benzodiazepin

1,93 g (4,97 Millimole) des nach Beispiel 16 hergestellten 8-[Chlor]-3-[äthoxycarbonyl]-4-[methyl]-1-[4'-(nitro)-phenyl]-4,5-di-[hydro]-3H-2,3-benzodiazepins werden in einer Mischung von Methanol und Dichlormethan im Volumverhältnis von 4 : 1 in Gegenwart eines Raney-Nickel-Katalysators mit Hydrazin in analoger Weise wie im Beispiel 2 reduziert. Das Produkt wird durch Säulenchromatographie (Silikagel, 1:1 n-Hexan/Äthylacetat-Mischung) gereinigt. Es werden 1,46 g der im Titel genannten Verbindung in Form eines festen Schaumes erhalten, Ausbeute 82%, F.: 95-98°C.

Beispiel 18

3-[n-Butylcarbamoyl]-8-[chlor]-4-[methyl]-1-[4'-(nitro)-phenyl]-4,5-di-[hydro]-3H-2,3-benzodiazepin

2,0 g (4,85 Millimole) 1-[<2'-(2''-{Hydroxy}-propyl)-5'-(chlor)-phenyl>-<4'''-(nitro)-phenyl>-methyliden]-4-[n-butyl]-semicarbazid werden in Dichlormethan in Gegenwart von 1,22 ml (8,73 Millimole) Triäthylamin mit 0,53 ml (6,79 Millimole) Mesylchlorid in analoger Weise zum Beispiel 14 acyliert. Das rohe Zwischenprodukt wird mit 0,28 ml (5,33 Millimole) einer 50 gew.-%-igen wäßrigen Natriumhydroxydlösung in Analogie zum Beispiel 14 cyclisiert. Das erhaltene Rohprodukt (1,40 g) wird aus Methanol umkristallisiert. Es werden 1,14 g der im Titel genannten Verbindung erhalten, Ausbeute 56%, F.: 150°C.

Das als Ausgangsstoff eingesetzte 1-[<2'-(2''-{Hydroxy}-propyl)-5'-(chlor)-phenyl>-<4'''-(nitro)-phenyl>-methyliden]-4-[n-butyl]-semicarbazid wird in Analogie zum Beispiel 14 aus 1-[Hydroxy]-7-[chlor]-3-[methyl]-1-[4'-(nitro)-phenyl]-isochroman (Beispiel 13) und 4-[n-Butyl]-semicarbazid hergestellt. Das in Form eines Harzes isolierte Rohprodukt wird nach Reinigung durch Säulenchromatographie (Silikagel; Hexan/Äthylacetat 1:1) als Ausgangsstoff eingesetzt. $R_F$ = etwa (0,2).

Beispiel 19

1-[4'-(Amino)-phenyl]-3-[n-butylcarbamoyl]-8-[chlor]-4-[methyl]-4,5-di-[hydro]-3H-2,3-benzodiazepin

1,14 g (2,75 Millimole) 1-[4'-(Nitro)-phenyl]-3-[n-butylcarbamoyl]-8-[chlor]-4-[methyl]-4,5-di-[hydro]-3H-2,3-benzodiazepin werden in 90 ml Methanol in Gegenwart eines Raney-Nickel-Katalysators mit 0,70 ml (13,74 Millimole) Hydrazinhydrat in Analogie zum Beispiel 2 reduziert. Das Produkt wird durch Säulenchromatographie gereinigt (Silikagel; Dichlormethan/Methanol = 98:2). Nach Einengen wird die im Titel genannte Verbindung in Form eines pulverisierbaren Schaumes isoliert. Ausbeute 0,93 g, 88%, F.: 89-91°C.

Beispiel 20

3-[Acetyl]-7,8-di-[chlor]-4-[methyl]-1-[4'-(nitro)-phenyl]-4,5-di-[hydro]-3H-2,3-benzodiazepin

(ein vom Beispiel 9 verschiedenes Verfahren)

6,26 g rohes Essigsäure-1-[<2'-(2''-{hydroxy}-propyl)-4',5'-di-(chlor)-phenyl>-<4'''-(nitro)-phenyl>-methyliden]-hydrazid (etwa 15,3 Millimole, Stufe B.) werden in Analogie zum Beispiel 13 mesyliert und durch Behandlung mit einer Base cyclisiert. Es werden 3,65 g der im Titel genannten Verbindung erhalten, Ausbeute 64% (auf das im Beispiel 9 A.) beschriebene Isochromanderivat bezogen). Das gelbe kristalline Rohprodukt schmilzt bei 190-195°C.

Der Ausgangsstoff wird auf die folgende Weise hergestellt:

A.) 1-[Hydroxy]-6,7-di-[chlor]-3-[methyl]-1-[4'-(nitro)-phenyl]-isochroman

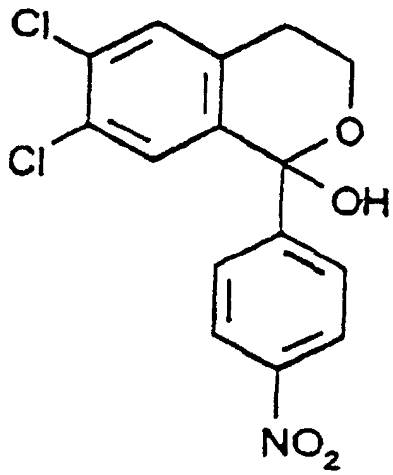

XIV

6, 0 g (17,7 Millimole) 6,7-Di-[chlor]-3-[methyl]-1-[4'-(nitro)-phenyl]-isochroman werden in Analogie zum Beispiel 13 A.) oxydiert. Es werden 6,03 g der im Titel genannten Verbindung erhalten, Ausbeute 96%. Das gelbe kristalline Rohprodukt ist ein Gemisch der beiden möglichen Stereoisomere und wird in der nächsten Stufe ohne weitere Reinigung eingesetzt.

B.) Essigsäure-1-[<2'-(2''-{hydroxy}-propyl)-4',5'-di-(chlor)-phenyl>-<4'''-(nitro)-phenyl>-methyliden]-hydrazid

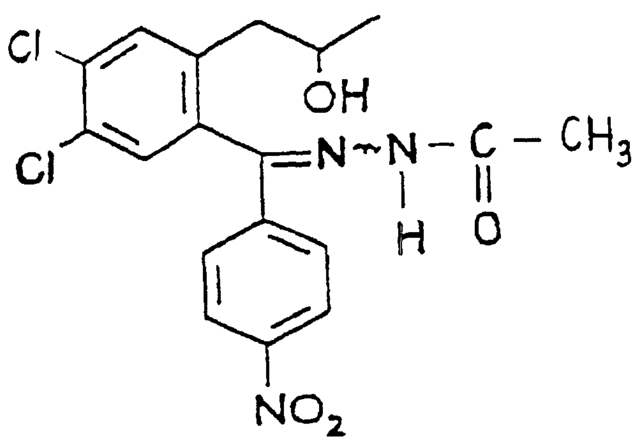

XV.

5,18 g (14,6 Millimole) des nach Stufe A.) hergestellten Hemiketals 1-[Hydroxy]-6,7-di-[chlor]-3-[methyl]-1-[4'-(nitro)-phenyl]-isochroman werden mit Essigsäurehydrazid in Analogie zum Beispiel 13 B.) umgesetzt. Das so erhaltene gelbe Harz wird in den darauffolgenden Mesylierungs- und Ringschlußreaktionen eingesetzt.

Beispiel 21

7,8-Di-[chlor]-4-[methyl]-3-[methylcarbamoyl]-1-[4'-(nitro)-phenyl]-4,5-di-[hydro]-3H-2,3-benzodiazepin

(ein vom Beispiel 11 verschiedenes Verfahren)

8,66 g (etwa 20,4 Millimole) rohes 1-[<2'-(2''-{Hydroxy}-propyl)-4',5'-di-(chlor)-phenyl>-<4'''-(nitro)-phenyl>-methyliden]-4-[methyl]-semicarbazid (wie unten beschrieben hergestellt) werden in Analogie zum Beispiel 13 mesyliert und durch basische Behandlung cyclisiert. Das erhaltene Rohprodukt wird aus wäßrigem Dimethylformamid umkristallisiert. Es werden 3,12 g der im Titel genannten Verbindung erhalten, Ausbeute 33% (auf das im Beispiel 9 A.) beschriebene Isochromanderivat bezogen). Die hellgelben Kristalle schmelzen bei 218-220°C.

1-[<2'-(2''-{Hydroxy}-propyl)-4',5'-di-(chlor)-phenyl>-<4'''-(nitro)-phenyl>-methyliden]-4-[methyl]-semicarbazid

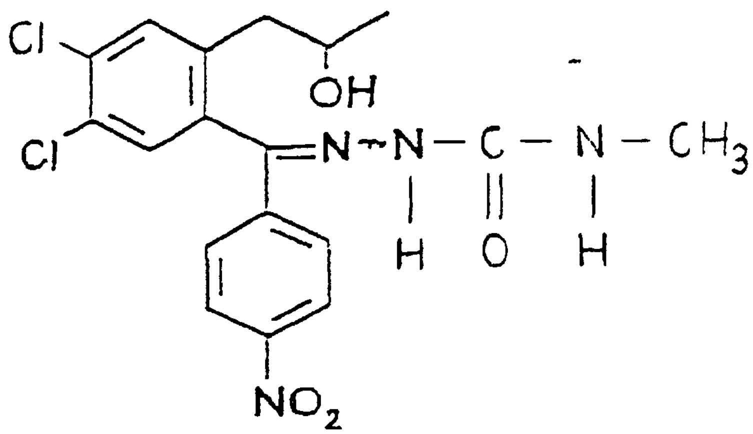

XVI.

Das als Ausgangsstoff eingesetzte 1-[<2'-(2''-{Hydroxy}-propyl)-4',5'-di-(chlor)-phenyl>-<4'''-(nitro)-phenyl>-methyliden]-4-[methyl]-semicarbazid wird in Analogie zum Beispiel 14 aus 1-[Hydroxy]-6,7-di-[chlor]-3-[methyl]-1-[4'-(nitro)-phenyl]-isochroman (Beispiel 20 A.) und 4-[Methyl]-semicarbazid hergestellt. Das als gelbes Harz isolierte Rohprodukt wird ohne weitere Reinigung weiterverarbeitet.

Beispiel 22

3-[Acetyl]-8-[brom]-4-[methyl]-1-[4'-(nitro)-phenyl]-4,5-di-[hydro]-3H-2,3-benzodiazepin

0,82 g (2, 28 Millimole) 8-[Brom]-4-[methyl]-1-[4'-(nitro)-phenyl]-4,5-di-[hydro]-3H-2,3-benzodiazepin werden in Analogie zum Beispiel 1 acetyliert. Es werden 0,84 g der im Titel genannten Verbindung erhalten, Ausbeute 91%; F.: 228-230°C.

Der Ausgangsstoff wird wie folgt hergestellt:

A.) 7-[Brom]-3-[methyl]-1-[4'-(nitro)-phenyl]-isochroman

Man verfährt wie im Beispiel 1 A.) mit dem Unterschied, daß man als Ausgangsstoff 14,69 g (68,3 Millimole) 1-[4'-(Brom)-phenyl]-2-isopropanol [J. Med. Chem. 21, 454 (1978)] verwendet. Es werden 10,94 g der im Titel genannten Verbindung erhalten, Ausbeute 46%, F.: 130-133°C.

B.) 7-[Brom]-3-[methyl]-1-[4'-(nitro)-phenyl]-2-benzopyrilium-perchlorat

10,8 g (32,5 Millimole) des nach Stufe A.) hergestellten Isochromanderivates werden in Analogie zum Beispiel 1 B.) mit Jones-Reagens oxydiert. Das Produkt wird in Eisessig als Medium durch Behandlung mit Perchlorsäure hergestellt. Es werden 4,91 g der im Titel genannten Verbindung erhalten, Ausbeute 34%, F.: 253-256°C.

C.) 8-[Brom]-4-[methyl]-1-[4'-(nitro)-phenyl]-5H-2,3-benzodiazepin

4,0 g (9,0 Millimole) des nach Stufe B.) hergestellten Benzopyrilium-perchlorats werden in analoger Weise wie im Beispiel 1 C.) mit Hydrazinhydrat umgesetzt. Es werden 2,30 g der im Titel genannten Verbindung erhalten, Ausbeute 71%, F.: 200-205°C.

D.) 8-[Brom]-4-[methyl]-1-[4'-(nitro)-phenyl]-4,5-di-[hydro]-3H-2,3-benzodiazepin

3,0 g (8,38 Millimole) des nach Stufe C.) hergestellten Benzodiazepinderivates werden in Analogie zum Beispiel 1 D.) reduziert. Es werden 2,79 g der im Titel genannten Verbindung erhalten. Ausbeute 92%, F.: 131-135°C.

Beispiel 23

3-[Acetyl]-1-[4'-(amino)-phenyl]-8-[brom]-4-[methyl]-4,5-di-[hydro]-3H-2,3-benzodiazepin

0,80 g (2, 0 Millimole) 3-[Acetyl]-1-[4'-(nitro)-phenyl]-8-[brom]-4-[methyl]-4,5-di-[hydro]-3H-2,3-benzodiazepin (Beispiel 22) werden in Analogie zum Beispiel 2 reduziert. Das Rohprodukt wird aus Äthanol kristallisiert. Es werden 0,62 g der im Titel genannten Verbindung erhalten, Ausbeute 83%, F.: 205-208°C.

Beispiel 24

8-[Brom]-4-[methyl]-1-[4'-(nitro)-phenyl]-3-[trifluoracetyl]-4,5-di-[hydro]-3H-2,3-benzodiazepin

1,51 g (4, 20 Millimole) 8-[Brom]-4-[methyl]-1-[4'-(nitro)-phenyl]-4,5-di-[hydro]-3H-2,3-benzodiazepin (Beispiel 22 D.) werden in 5 ml Dichlormethan und 5 ml Trifluoressigsäureanhydrid bei 25°C 3 Stunden lang gerührt. Das Reaktionsgemisch wird mit Dichlormethan verdünnt. Die organische Phase wird mit Wasser und einer 2 gew.-%-igen Natriumbicarbonatlösung gewaschen, getrocknet und eingeengt. Das Rohprodukt wird durch Suspendieren in 5 ml heißem Äthanol gereinigt. Nach Filtrieren und Trocknen werden 2,66 g der im Titel genannten Verbindung erhalten, Ausbeute 86%, F.: 193-196°C.

Beispiel 25

1-[4'-(Amino)-phenyl]-8-[brom]-4-[methyl]-3-[trifluoracetyl]-4,5-di-[hydro]-3H-2,3-benzodiazepin

1, 64 g (3,60 Millimole) 1-[4'-(Nitro)-phenyl]-8-[brom]-4-[methyl]-3-[trifluoracetyl]-4,5-di-[hydro]-3H-2,3-benzodiazepin (Beispiel 24) werden in Analogie zum Beispiel 2 reduziert. Das Rohprodukt wird durch Chromatographie (Silikagelsäule) und Eluieren (Chloroform/Methanol 99:1) gereinigt. Es werden 1,33 g der im Titel genannten Verbindung erhalten, Ausbeute 87%, F.: 93-96°C.

Beispiel 26

8-[Brom]-4-[methyl]-1-[4'-(nitro)-phenyl]-3-[propionyl]-4,5-di-[hydro]-3H-2,3-beazodiazepin

1,33 g (3,7 Millimole) 8-[Brom]-4-[methyl]-1-[4'-(nitro)-phenyl]-4,5-di-[hydro]-3H-2,3-benzodiazepin (Beispiel 22 D.) werden in Analogie zum Beispiel 1 acyliert, mit dem Unterschied, daß als Acylierungsmittel Propionsäureanhydrid verwendet wird. Das Rohprodukt wird durch Chromatographie (Silikagelsäule) und Eluieren (Hexan/Äthylacetat 9:1) gereinigt. Das Propionsäureanhydrid-Handelsprodukt enthält einige Prozente Essigsäureanhydrid. Deshalb ist das Produkt durch das Acetylderivat verunreinigt; dieses Nebenprodukt kann durch Chromatographie entfernt werden. Es werden 1,07 g der im Titel genannten Verbindung erhalten, Ausbeute 70%, F.: 178-180°C.

Beispiel 27

1-[4'-(Amino)-phenyl]-8-[brom]-4-[methyl]-3-[propionyl]-4,5-di-[hydro]-3H-2,3-benzodiazepin

1, 05 g (2,52 Millimole) 1-[4'-(Nitro)-phenyl]-8-[brom]-4-[methyl]-3-[propionyl]-4,5-di-[hydro]-3H-2,3-benzodiazepin (Beispiel 26) werden in Analogie zum Beispiel 2 reduziert. Das Rohprodukt wird aus Äthanol kristallisiert. Es werden 0,85 g der im Titel genannten Verbindung erhalten, Ausbeute 87%, F.: 99-102°C.

Beispiel 28

8-[Brom]-3-[cyclopropionyl]-4-[methyl]-1-[4'-(nitro)-phenyl]-4,5-di-[hydro]-3H-2,3-benzodiazepin

0,90 g (2,5 Millimole) 8-[Brom]-4-[methyl]-1-[4'-(nitro)-phenyl]-4,5-di-[hydro]-3H-2,3-benzodiazepin (Beispiel 22 D.) werden in 15 ml wasserfreiem Dichlormethan in Gegenwart von 0,22 ml (3 Millimole) Triäthylamin bei 25°C mit 0,27 ml (3 Millimole) Cyclopropansäurechlorid acyliert. Das Reaktionsgemisch wird auf 25 g zerstoßenes Eis gegossen und mit Dichlormethan extrahiert. Die organische Phase wird mit Wasser und einer Natriumcarbonatlösung gewaschen, getrocknet und eingedampft. Das Rohprodukt wird in 3 ml heißem Äthanol suspendiert, filtriert und getrocknet. Es werden 0,88 g der im Titel genannten Verbindung erhalten, Ausbeute 82%, F.: 172-173°C.

Beispiel 29

1-[4'-(Amino)-phenyl]-8-[brom]-3-[cyclopropionyl]-4-[methyl]-4,5-di-[hydro]-3H-2,3-benzodiazepin

0,76 g (1,77 Millimole) 1-[4'-(Nitro)-phenyl]-8-[brom]-3-[cyclopropionyl]-4-[methyl)-4,5-di-[hydro]-3H-2,3-benzodiapezin (Beispiel 28) werden in Analogie zum Beispiel 2 reduziert. Das Rohprodukt wird aus einem Äthanol/Hexan 1:4 Gemisch kristallisiert. Es werden 0,55 g der im Titel genannten Verbindung erhalten, Ausbeute 78%, F.: 113-116°C.

Beispiel 30

8-[Brom]-4-[methyl]-3-[methylcarbamoyl]-1-[4'-(nitro)-phenyl]-4,5-di-[hydro]-3H-2,3-benzodiazepin

0,84 g (2,33 Millimole) 8-[Brom]-4-[methyl]-1-[4'-(nitro)-phenyl]-4,5-di-[hydro]-3H-2,3-benzodiazepin (Beispiel 22 D.) werden in Analogie zum Beispiel 3 mit Methylisocyanat umgesetzt. Das Rohprodukt wird in 5 ml heißem Äthanol suspendiert. Nach Filtrieren und Trocknen wird 0,82 g der im Titel genannten Verbindung erhalten, Ausbeute 84%, F.: 197-200°C.

Beispiel 31

1-[4'-(Amino)-phenyl]-8-[brom]-4-[methyl]-3-[methylcarbamoyl]-4,5-di-[hydro]-3H-2,3-benzodiazepin

0,80 g (1,92 Millimole) 1-[4'-(Nitro)-phenyl]-8-[brom]-4-[methyl]-3-[methylcarbamoyl]-4,5-di-[hydro]-3H-2,3-benzodiazepin (Beispiel 30) werden in Analogie zum Beispiel 2 reduziert. Das Rohprodukt wird aus 3 ml Äthylacetat kristallisiert. Es werden 0,50 g der im Titel genannten Verbindung erhalten, Ausbeute 68%, F.: 121-124°C.

Beispiel 32

8-[Brom]-3-[äthoxycarbonyl]-4-[methyl]-1-[4'-(nitro)-phenyl]-4,5-di-[hydro]-3H-2,3-benzodiazepin

8,11 g (18 Millimole) 3-[<2'-(2''-{hydroxy}-propyl)-5'-(brom)-phenyl>-<4'-(nitro)-phenyl>-methyliden]-äthylcarbazat werden in Analogie zum Beispiel 16 zuerst mit Mesylchlorid und darauffolgend mit einer 50 gew.-%-igen Natriumhydroxydlösung umgesetzt. Das Produkt wird mit Dichlormethan extrahiert, die organische Phase mit Wasser gewaschen, getrocknet und eingeengt. Das erhaltene Produkt (6,78 g, 87%) ist ein fester Schaum und wird im nächsten Beispiel in dieser Form verwendet.

Der Ausgangsstoff wird wie folgt hergestellt:

A.) 7-[Brom]-1-[hydroxy]-3-[methyl]-1-[4'-(nitro)-phenyl]-isochroman

15,56 g (44,7 Millimole) 7-[Brom]-3-[methyl]-1-[4'-(nitro)-phenyl]-isochroman (Beispiel 22 A.) werden in Analogie zum Beispiel 13 A.) oxydiert. Das Produkt wird mit Benzol extrahiert, getrocknet und eingeengt. Es werden 14,80 g der im Titel genannten Verbindung in Form eines Isomerengemisches erhalten, Ausbeute 91%. Das Produkt wird ohne weitere Reinigung weiterverarbeitet.

B.) 3-[<2'-(2''-{Hydroxy}-propyl)-5'-(brom)-phenyl>-<4'-(nitro)-phenyl>-methyliden]-äthylcarbazat

Das nach Stufe A hergestellte Hemiketalderivat 7-[Brom]-1-[hydroxy]-3-[methyl]-1-[4'-(nitro)-phenyl]-isochroman (6,82 g, 18,72 Millimole) wird in Analogie zum Beispiel 16 mit Äthylcarbazat umgesetzt. Das Produkt wird mit

Äthylacetat extrahiert, die organische Phase mit Wasser gewaschen, getrocknet und eingeengt. Es werden 8,11 g der im Titel genannten Verbindung erhalten, Ausbeute 96%. Das Produkt ist ein Isomergemisch und wird ohne weitere Reinigung weiterverarbeitet.

Beispiel 33

1-[4'-(Amino)-phenyl]-8-[brom]-3-[äthoxycarbonyl]-4-[methyl]-4,5-di-[hydro]-3H-2,3-benzodiazepin

1,58 g (3, 66 Millimole) 1-[4'-(Nitro)-phenyl]-8-[brom]-3-[äthoxycarbonyl]-4-[methyl]-4,5-di-[hydro]-3H-2,3-benzodiazepin (Beispiel 32) werden in Analogie zum Beispiel 2 reduziert. Das Produkt wird aus 4 ml Äthylacetat kristallisiert. Es werden 1,20 g der im Titel genannten Verbindung erhalten, Ausbeute 81%, F.: 114-117°C.

Beispiel 34

8-[Brom]-3-[carbamoyl]-4-[methyl]-1-[4'-(nitro)-phenyl]-4,5-di-[hydro]-3H-2,3-benzodiazepin

1,80 g (5 Millimole) 8-[Brom]-4-[methyl]-1-[4'-(nitro)-phenyl]-4,5-di-[hydro]-3H-2,3-benzodiazepin werden in 10 ml Eisessig mit 0,53 g (6,5 Millimole) Kaliumcyanat umgesetzt. Nach einer Stunde wird das Reaktionsgemisch auf 1 ml Wasser gegossen. Die ausgeschiedenen Kristalle werden abfiltriert. Das Rohprodukt wird in 15 ml heißem Äthanol suspendiert. Nach Filtrieren und Trocknen werden 1,56 g der im Titel genannten Verbindung erhalten, Ausbeute 77%, F.: 193-203°C.

Der Ausgangsstoff wird wie folgt hergestellt:

8-[Brom]-4-[methyl]-1-[4'-(nitro)-phenyl]-4,5-di-[hydro]-3H-2,3-benzodiazepin (Verbindung nach Beispiel 22 D.)

5,20 g (12 Millimole) 8-[Brom]-3-[äthoxycarbonyl]-4-[methyl]-1-[4'-(nitro)-phenyl]-4,5-di-[hydro]-3H-2,3-benzodiazepin (Beispiel 32) werden in 104 ml Methanol mit 6 ml einer 10 N Natriumhydroxydlösung 2 Stunden lang zum Sieden erhitzt. Das Reaktionsgemisch wird abgekühlt, mit 104 ml Wasser verdünnt und die ausgeschiedenen Kristalle werden abfiltriert. Es werden 3,99 g der im Titel genannten Verbindung erhalten, Ausbeute 92%, F.: 125-130°C.

Beispiel 35

1-[4'-(Amino)-phenyl]-8-[brom]-3-[carbamoyl]-4-[methyl]-4,5-di-[hydro]-3H-2,3-benzodiazepin

1,55 g (3, 84 Millimole) 1-[4'-(Nitro)-phenyl]-8-[brom]-3-[carbamoyl]-4-[methyl]-4,5-di-[hydro]-3H-2,3-benzodiazepin (Beispiel 34) werden in Analogie zum Beispiel 2 reduziert. Das Rohprodukt wird aus 5 ml Äthanol kristallisiert. Es werden 1,19 g der im Titel genannten Verbindung erhalten, Ausbeute 83%, F.: 218-221°C.

Beispiel 36

8-[Chlor]-4-[methyl]-1-[4'-(nitro)-phenyl]-3-[trifluoracetyl]-4,5-di-[hydro]-3H-2,3-benzodiazepin

0,6 g (1,9 Millimole) 8-[Chlor]-4-[methyl]-1-[4'-(nitro)-phenyl]-4,5-di-[hydro]-3H-2,3-benzodiazepin (Beispiel 5 D.) werden in Analogie zum Beispiel 24 mit Trifluoressigsäureanhydrid acyliert. Es werden 0,76 g der im Titel genannten Verbindung erhalten, Ausbeute 97%, F.: 150-152°C.

Der Ausgangsstoff ist mit dem Produkt des Beispieles 5 D.) identisch und kann auch auf die folgende Weise hergestellt werden:

10,98 g (27 Millimole) 3-[Äthoxycarbonyl]-8-[chlor]-4-[methyl]-1-[4'-(nitro)-phenyl]-4,5-di-[hydro]-3H-2,3-benzodiazepin (Beispiel 16) werden in Analogie zum Beispiel 34 hydrolysiert. Es werden 8,04 g der gewünschten Verbindung erhalten, Ausbeute 94%, F.: 146-151°C.

Beispiel 37

1-[4'-(Amino)-phenyl]-8-[chlor]-4-[methyl]-3-[trifluoracetyl]-4,5-di-[hydro]-3H-2,3-benzodiazepin

0,75 g (1,8 Millimole) 1-[4'-(Nitro)-phenyl]-8-[chlor]-4-[methyl]-3-[trifluoracetyl]-4,5-di-[hydro]-3H-2,3-benzodiazepin (Beispiel 36) werden in Analogie zum Beispiel 2 reduziert. Das Rohprodukt wird durch Chromatographie (Kieselgelsäule) und Eluieren (Benzol/Äthylacetat 3:1) gereinigt. Es werden 0,47 g der im Titel genannten Verbindung

erhalten, Ausbeute 68%, F.: 165-167°C.

Beispiel 38

8-[Chlor]-4-[methyl]-1-[4'-(nitro)-phenyl]-3-[propionyl]-4,5-di-[hydro]-3H-2,3-benzodiazepin

0, 6 g (1,9 Millimole) 8-[Chlor]-4-[methyl]-1-[4'-(nitro)-phenyl]-4,5-di-[hydro]-3H-2,3-benzodiazepin (Beispiel 5 D. oder Ausgangsstoff von Beispiel 36) werden in Analogie zum Beispiel 26 mit Propionsäureanhydrid acyliert. Das Rohprodukt wird durch Chromatographie (Silikagelsäule) und Eluieren (Benzol/Äthylacetat 95:5) gereinigt. Es werden 0,56 g der im Titel genannten Verbindung erhalten, Ausbeute 79%, F.: 160-161°C.

Beispiel 39

1-[4'-(Amino)-phenyl]-8-[chlor]-4-[methyl]-3-[propionyl]-4,5-di-[hydro]-3H-2,3-benzodiazepin

0,46 g (1,23 Millimole) 1-[4'-(Nitro)-phenyl]-8-[chlor]-4-[methyl]-3-[propionyl]-4,5-di-[hydro]-3H-2,3-benzodiazepin (Beispiel 38) werden in Analogie zum Beispiel 2 reduziert. Das Rohprodukt wird aus 50 gew.-%-igem wäßrigem Äthanol kristallisiert. Es werden 0,39 g der im Titel genannten Verbindung erhalten, Ausbeute 93%, F.: 118-120°C.

Beispiel 40

3-[Cyclopropionyl]-8-[chlor]-4-[methyl]-1-[4'-(nitro)-phenyl]-4,5-di-[hydro]-3H-2,3-benzodiazepin

0, 6 g (1,9 Millimole) 8-[Chlor]-4-[methyl]-1-[4'-(nitro)-phenyl]-4,5-di-[hydro]-3H-2,3-benzodiazepin (Beispiel 5 D. oder Ausgangsstoff von Beispiel 36) werden in Analogie zum Beispiel 28 mit Cyclopropancarbonsäurechlorid acyliert. Es werden 0,71 g der im Titel genannten Verbindung erhalten, Ausbeute 97%, F.: 158-160°C.

Beispiel 41

1-[4'-(Amino)-phenyl]-3-[cyclopropionyl]-8-[chlor]-4-[methyl]-4,5-di-[hydro]-3H-2,3-benzodiazepin

0,72 g (1,87 Millimole) 1-[4'-(Nitro)-phenyl]-3-[cyclopropionyl]-8-[chlor]-4-[methyl]-4,5-di-[hydro]-3H-2,3-benzodiazepin (Beispiel 40) werden in Analogie zum Beispiel 2 reduziert. Das Rohprodukt wird aus 50 gew.-%-igem wäßrigem Äthanol umkristallisiert. Es werden 0,57 g der im Titel genannten Verbindung erhalten, Ausbeute 86%, F.: 122-124°C.

Beispiel 42

(+)-3-[Acetyl]-8-[chlor]-4-[methyl]-1-[4'-(nitro)-phenyl]-4,5-di-[hydro]-5H-2,3-benzodiazepin

0,51 g (1, 6 Millimole) (+)-8-[Chlor]-4-[methyl]-1-[4'-(nitro)-phenyl]-4,5-di-[hydro]-5H-2,3-benzodiazepin werden mit Essigsäureanhydrid in Analogie zum Beispiel 1 acetyliert. Das Rohprodukt (0,54 g) wird aus 28 ml Äthylacetat bei Raumtemperatur kristallisiert. Es werden 0,28 g der im Titel genannten Verbindung erhalten, Ausbeute 48%, F.: 259-260°C.

Nach chiraler HPLC-Analyse (CHIRALCEL OJ®; Eluiermittel: Hexan/Äthylacetat 1:1) ist das Produkt stereoeinheitlich.

Der Ausgangsstoff wird wie folgt hergestellt:

(+)-8-[Chlor]-4-[methyl]-1-[4'-(nitro)-phenyl]-4,5-di-[hydro]-5H-2,3-benzodiazepin

Einer Lösung von 6,03 g (23,6 Millimole) R-(+)-2-[Amino]-1,1-di-[phenyl]-3-[methyl]-butan-1-ol [J. Org. Chem. 49, 555 (1984); J. Chem. Soc. Perkin Trans I. 2039 (1985)] und 60 ml wasserfreiem Dichloräthan werden bei -20°C 2,43 ml (21,5 Millimole) eines Boran/Dimethylsulfid-Komplexes tropfenweise zugegeben (Borankonzentration etwa 9,2 M). Die Temperatur der Lösung wird innerhalb von 3 Stunden auf 0°C erhöht und 15 Stunden lang auf +4°C gehalten. Der so erhaltenen reduzierenden Komplexlösung wird bei Raumtemperatur während 30 Minuten eine Lösung von 3,37 g (10,7 Millimole) 8-[Chlor]-4-[methyl]-1-[4'-(nitro)-phenyl]-4,5-di-[hydro]-5H-2,3-benzodiazepin (Beispiel 5 C.) und 60 ml wasserfreiem Dichloräthan tropfenweise zugegeben. Das Reaktonsgemisch wird bei 60°C 6 Stunden lang gerührt. Die orangegelbe Lösung wird auf 25°C gekühlt, mit 50 ml einer 10 gew.-%-igen Natriumcarbonatlösung

behandelt, mit Wasser neutral gewaschen, getrocknet und eingeengt. Das Produkt wird auf einer Silikagelsäule durch Eluieren mit einem Benzol/Äthylacetat 8:1 Gemisch isoliert. Das so erhaltene Produkt ist ein 75:25 Enantiomerengemisch (HPLC: Chiralcel OJ®, Eluiermittel: Hexan-Äthanol 1:1).

Beispiel 43

(-)-3-[Acetyl]-1-[4'-(amino)-phenyl]-8-[chlor]-4-[methyl]-4,5-di-[hydro]-5H-2,3-benzodiazepin

0,28 g (0,78 Millimole) (+)-3-Acetyl-1-[4'-(nitro)-phenyl]-8-[chlor]-4-[methyl]-4,5-di-[hydro]-5H-2,3-benzodiazepin (Beispiel 42) werden in Analogie zum Beispiel 2 reduziert. Das Rohprodukt wird aus 2 ml Äthanol kristallisiert. Es werden 0,15 g der im Titel genannten Verbindung erhalten, Ausbeute 58%, F.: 219-220°C.
$[\alpha]_D^{20}$ = -712,1° (c = 0,7, Chloroform). Das Rohprodukt ist ein einheitliches Enantiomer (HPLC: Chiralcel OJ®, Eluiermittel Hexan-Äthanol = 1:1).

Beispiel 44

3-[Äthylcarbamoyl]-8-[chlor]-4-[methyl]-1-[4'-(nitro)-phenyl]-4,5-di-[hydro]-3H-2,3-benzodiazepin

0, 6 g (1,9 Millimole) 8-[Chlor]-4-[methyl]-1-[4'-(nitro)-phenyl]-4,5-di-[hydro]-3H-2,3-benzodiazepin (Beispiel 5 D. oder Ausgangsstoff von Beispiel 36) wird in 25 ml wasserfreiem Toluol mit 0,75 ml (9,5 Millimole) Äthylisocyanat 24 Stunden lang zum Sieden erhitzt. Das Reaktionsgemisch wird eingeengt und der Rückstand aus 5 ml Äthanol umkristallisiert. Es werden 0,51 g der im Titel genannten Verbindung erhalten, Ausbeute 69%, F.: 170-172°C.

Beispiel 45

1-[4'-(Amino)-phenyl]-3-[äthylcarbamoyl]-8-[chlor]-4-[methyl]-4,5-di-[hydro]-3H-2,3-benzodiazepin

0,48 g (1, 24 Millimole) 1-[4'-(Nitro)-phenyl]-3-[äthylcarbamoyl]-8-[chlor]-4-[methyl]-4,5-di-[hydro]-3H-2,3-benzodiazepin (Beispiel 44) werden in Analogie zum Beispiel 2 reduziert. Das Rohprodukt wird aus 4 ml 50 gew.-%-igem wäßrigem Äthanol umkristallisiert. Es werden 0,35 g der im Titel genannten Verbindung erhalten, Ausbeute 79%, F.: 165-167°C.

Beispiel 46

8-[Chlor]-4-[methyl]-1-[4'-(nitro)-phenyl]-3-[n-propylcarbamoyl]-4,5-di-[hydro]-3H-2,3-benzodiazepin

0,6 g (1,9 Millimole) 8-[Chlor]-4-[methyl]-1-[4'-(nitro)-phenyl]-4,5-di-[hydro]-3H-2,3-benzodiazepin (Beispiel 5 D. oder Ausgangsstoff des Beispieles 36) werden mit n-Propylisocyanat in Analogie zum Beispiel 44 umgesetzt. Das Rohprodukt wird aus Äthanol umkristallisiert. Es werden 0,63 g der im Titel genannten Verbindung erhalten, Ausbeute 83%, F.: 186-187°C.

Beispiel 47

1-[4'-(Amino)-phenyl]-8-[chlor]-4-[methyl]-3-[n-propylcarbamoyl]-4,5-di-[hydro]-3H-2,3-benzodiazepin

0,6 g (1,5 Millimole) 1-[4'-(Nitro)-phenyl]-8-[chlor]-4-[methyl]-3-[n-propylcarbamoyl]-4,5-di-[hydro]-3H-2,3-benzodiazepin (Beispiel 46) werden in Analogie zum Beispiel 2 reduziert. Es werden 0,52 g der im Titel genannten Verbindung erhalten, Ausbeute 93%, F.: 88-90°C.

Beispiel 48

3-[Isopropylcarbamoyl]-8-[chlor]-4-[methyl]-1-[4'-(nitro)-phenyl]-4,5-di-[hydro]-3H-2,3-benzodiazepin

0, 6 g (1,9 Millimole) 8-[Chlor]-4-[methyl]-1-[4'-(nitro)-phenyl]-4,5-di-[hydro]-3H-2,3-benzodiazepin (Beispiel 5 D. oder Ausgangsstoff des Beispieles 36) werden in Analogie zum Beispiel 44 mit Isopropylisocyanat umgesetzt. Das Rohprodukt wird aus Äthanol umkristallisiert. Es werden 0,4 g der im Titel genannten Verbindung erhalten, Ausbeute 53%, F.: 172-174°C.

Beispiel 49

1-[4'-(Amino)-phenyl]-3-[isopropylcarbamoyl]-8-[chlor]-4-[methyl]-4,5-di-[hydro]-3H-2,3-benzodiazepin

0,38 g (0,95 Millimole) 1-[4'-(Nitro)-phenyl]-3-[isopropylcarbamoyl]-8-[chlor]-4-[methyl]-4,5-di-[hydro]-3H-2,3-benzodiazepin (Beispiel 48) werden in Analogie zum Beispiel 2 reduziert. Es werden 0,32 g der im Titel genannten Verbindung erhalten, Ausbeute 91%, F.: 100-102°C.

Beispiel 50

3-[Äthoxycarbonyl]-7,8-di-[chlor]-4-[methyl]-1-[4'-(nitro)-phenyl]-4,5-di-[hydro]-3H-2,3-benzodiazepin

1,41 g (etwa 2,7 Millimole) rohes 3-[<2'-(2''-{Hydroxy}-propyl)-4',5'-di-(chlor)-phenyl>-<4'-(nitro)-phenyl>-methyliden]-äthylcarbazat werden in Analogie zum Beispiel 13 mesyliert und durch Behandlung mit einer Base cyclisiert. Es werden 0,94 g der im Titel genannten Verbindung erhalten, Ausbeute 75% (auf das im Beispiel 9 A. beschriebene Isochromanderivat bezogen), F.: 106-108°C.

Der Ausgangsstoff wird wie folgt hergestellt:

3-[<2'-(2''-{Hydroxy}-propyl)-4',5'-di-(chlor)-phenyl>-<4'-(nitro)-phenyl>-methyliden]-äthylcarbazat

0,98 g (2, 8 Millimole) 1-[Hydroxy]-6,7-di-[chlor]-3-[methyl]-1-[4'-(nitro)-phenyl]-isochroman (Beispiel 20 A.) werden in Analogie zum Beispiel 16 mit Äthylcarbazat umgesetzt. Es werden 1,20 g der im Titel genannten Verbindung erhalten, Ausbeute 97%. Das Produkt ist ein Isomerengemisch und wird ohne weitere Reinigung weiterverarbeitet.

Beispiel 51

1-[4'-(Amino)-phenyl]-3-[äthoxycarbonyl]-7,8-di-[chlor]-4-[methyl]-4,5-di-[hydro]-3H-2,3-benzodiazepin

0,94 g (2,2 Millimole) 1-[4'-(Nitro)-phenyl]-3-[äthoxycarbonyl]-7,8-di-[chlor]-4-[methyl]-4,5-di-[hydro]-3H-2,3-benzodiazepin (Beispiel 50) werden in Analogie zum Beispiel 2 reduziert. Das Rohprodukt wird durch Chromatographie (Silikagelsäule) und Eluieren (Benzol/Äthylacetat 4:1) gereinigt und aus Äthanol umkristallisiert. Es werden 0,54 g der im Titel genannten Verbindung erhalten, Ausbeute 63%, F.: 190-192°C.

Beispiel 52

3-[Butyryl]-7,8-di-[chlor]-4-[methyl]-1-[4'-(nitro)-phenyl]-4,5-di-[hydro]-3H-2,3-benzodiazepin

1,31 g (etwa 3 Millimole) rohes Buttersäure-1-[<2'-(2''-{Hydroxy}-propyl)-4',5'-di-(chlor)-phenyl>-<4'''-(nitro)-phenyl>-methyliden]-hydrazid werden in Analogie zum Beispiel 13 mesyliert und durch Behandlung mit einer Base cyclisiert. Es werden 1,08 g der im Titel genannten Verbindung erhalten, Ausbeute 86% (auf das im Beispiel 9 A.) beschriebene Isochromanderivat bezogen), F.: 75-77°C.

Der Ausgangsstoff wird wie folgt hergestellt:

Buttersäure-1-[<2'-(2''-{Hydroxy}-propyl)-4',5'-di-(chlor)-phenyl>-<4'''-(nitro)-phenyl>-methyliden]-hydrazid

10,6 g (3 Millimole) des nach Beispiel 20 A.) hergestellten Hemiketals 1-[Hydroxy]-6,7-di-[chlor]-3-[methyl]-1-[4'-(nitro)-phenyl]-isochroman werden in Analogie zum Beispiel 13 B.) mit Buttersäurehydrazid umgesetzt. Das so erhaltene gelbe amorphe Produkt (1,31 g) wird bei der Mesylierungs- und Cyclisierungsreaktion ohne weitere Reinigung eingesetzt.

Beispiel 53

1-[4'-(Amino)-phenyl]-3-[butyryl]-7,8-di-[chlor]-4-[methyl]-4,5-di-[hydro]-3H-2,3-benzodiazepin

1,08 g (2, 6 Millimole) 1-[4'-(Nitro)-phenyl]-3-[butyryl]-7,8-di-[chlor]-4-[methyl]-4,5-di-[hydro]-3H-2,3-benzodiazepin (Beispiel 52) werden in Analogie zum Beispiel 2 reduziert. Das Rohprodukt wird durch Chromatographie (Silikagelsäule) und Eluieren (Benzol-Äthylacetat 8:1) gereinigt und in heißem Äthanol suspendiert. Es werden 0,57 g der im Titel genannten Verbindung erhalten, Ausbeute 57%, F.: 193-194°C.

In analoger Weise wie in den vorherigen Beispielen werden die folgenden Verbindungen hergestellt:

1-[4'-(Nitro)-phenyl]-7,8-di-[chlor]-4-[methyl]-3-[propionyl]-4,5-di-[hydro]-3H-2,3-benzodiazepin

1-[4'-(Amino)-phenyl]-7,8-di-[chlor]-4-[methyl]-3-[propionyl]-4,5-di-[hydro]-3H-2,3-benzodiazepin

1-[4'-(Nitro)-phenyl]-3-[carbamoyl]-7,8-di-[chlor]-4-[methyl]-4,5-di-[hydro]-3H-2,3-benzodiazepin

1-[4'-(Amino)-phenyl]-3-[carbamoyl]-7,8-di-[chlor]-4-[methyl]-4,5-di-[hydro]-3H-2,3-benzodiazepin.

## Patentansprüche

**1.** 7- und/oder 8-Halogensubstituierte 2,3-Benzodiazepinderivate der allgemeinen Formel

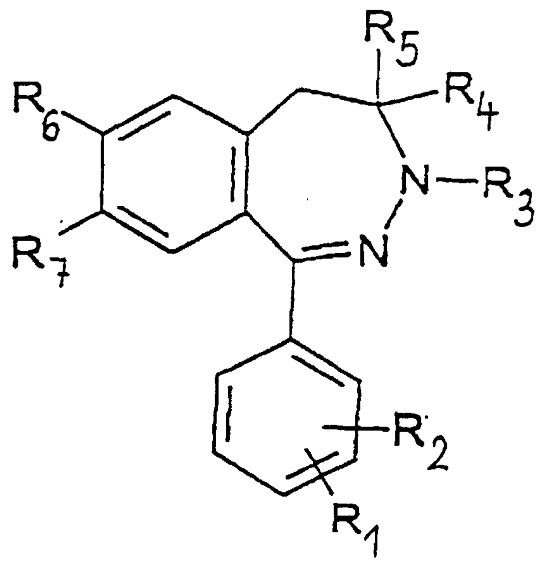

I,

worin

$R_1$ und $R_2$ unabhängig voneinander für Wasserstoff- bzw. Halogenatome beziehungsweise $C_{1-4}$-Alkyl-, $C_{1-4}$-Alkoxy-, Nitro- bzw. Trifluormethylgruppen bzw. einen Aminorest der allgemeinen Formel

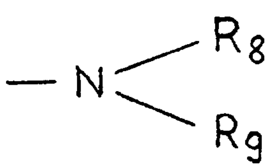

II

stehen,
in welchletzterer

$R_8$ und $R_9$ unabhängig voneinander Wasserstoffatome, $C_{1-4}$-Alkylreste bzw. Reste der allgemeinen Formel

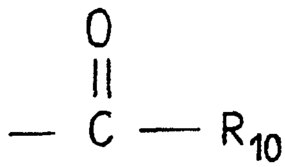

III

bedeuten,

in welchletzterer

$R_{10}$ ein Wasserstoffatom bzw. einen, gegebenenfalls durch 1 oder mehr $C_{1-4}$-Alkoxyrest(e) und/oder Halogenatom(e) substituierten, $C_{1-6}$-Alkyl-, $C_{6-10}$-Aryl-, $C_{1-4}$-Alkoxy-, $C_{3-5}$-Cycloalkyl-, $C_{2-6}$-Alkenyl- bzw. $C_{3-5}$-Cycloalkoxyrest bzw. Aminorest der allgemeinen Formel

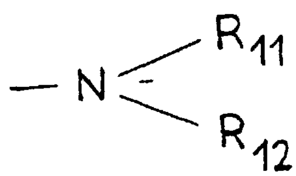

IV

darstellt,

in welchletzterer

$R_{11}$ und $R_{12}$ unabhängig von- einander Wasserstoffatome bzw. $C_{1-4}$-Alkyl-, $C_{3-5}$-Cycloalkyl- bzw. $C_{6-10}$-Aryl-reste sind,

$R_3$ einen $C_{1-4}$-Alkyl- oder $C_{3-5}$-Cycloalkylrest bzw. einen Rest der allgemeinen Formel

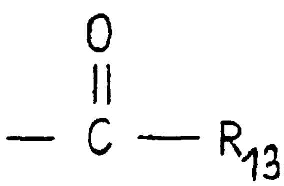

V

bedeutet,

in welchletzterer

$R_{13}$ dieselben Bedeutungen wie $R_{10}$ hat,

$R_4$ und $R_5$ unabhängig voneinander Wasserstoff bzw. $C_{1-3}$-Alkyl-reste darstellen

$R_6$ und $R_7$ unabhängig voneinander für Wasserstoff-, Chlor-bzw. Bromatome stehen,

mit der weiteren Maßgabe, daß

mindestens 1 von

$R_6$ und $R_7$ von Wasserstoff verschieden ist,

sowie deren Isomere und die Säureadditionssalze dieser Verbindungen.

**2.** 2,3-Benzodiazepinderivate nach Anspruch 1, **dadurch gekennzeichnet, daß** der/die Alkylrest(e), für welche[n] $R_1$ , $R_2$ , $R_8$ , $R_9$ , $R_{11}$ , $R_{12}$ und/oder $R_3$ stehen kann/können, [ein] solche[r] mit 1 bis 3, insbesondere 1 oder 2, Kohlenstoffatom(en) ist/sind, der/die Alkoxyrest(e), welche [n] $R_1$ , $R_2$ , $R_{10}$ und/oder $R_{13}$ bedeuten kann/können und/oder durch welche[n] die $C_{1-6}$-Alkyl-, $C_{6-10}$-Aryl-, $C_{1-4}$-Alkoxy-, $C_{3-5}$-Cycloalkyl-, $C_{2-6}$-Alkenyl- bzw. $C_{3-5}$-Cyclo-

alkoxyreste, für die $R_{10}$ und oder $R_{13}$ stehen kann/können, substituiert sein kann/können, [ein] solche[r] mit 1 bis 3, insbesondere 1 oder 2, Kohlenstoffatom(en) ist/sind, der/die Alkylrest(e), für welchen $R_{10}$ und/oder $R_{13}$ stehen kann/können, [ein] solche[r] mit 1 bis 4, insbesondere 1 bis 3, Kohlenstoffatom(en) ist/sind, der/die Alkylrest(e), für welche[n] $R_4$ und/oder $R_5$ stehen kann/können, [ein] solche[r] mit 1 oder 2, insbesondere 1, Kohlenstoffatom (en) ist/sind, der/die Alkenylrest(e), für welche[n] $R_{10}$ und/oder $R_{13}$ stehen kann/können, [ein] solche[r] mit 2 bis 4, insbesondere 2 oder 3, Kohlenstoffatomen ist/sind, der/die Cycloalkyl- oder Cycloalkoxyrest(e), welche[n] $R_{10}$ und/oder $R_{13}$ bedeuten kann/können und/oder der/die Cycloalkylrest(e), welche[n] $R_3$ , $R_{11}$ und/oder $R_{12}$ darstellen kann/können, [ein] solche[r] mit 3 oder 4, insbesondere 3, Kohlenstoffatomen ist/sind, die/die Arylrest(e), für welche [n] $R_{10}$ , $R_{13}$ , $R_{11}$ und/oder $R_{12}$ stehen kann/können, [ein] Phenyl- und/oder Naphthylrest(e) ist/sind und/oder das/die Halogenatom(e), für welche[s] $R_1$ und/oder $R_2$ stehen kann/können und/oder durch welche[s] die $C_{1-6}$-Alkyl-, $C_{6-10}$-Aryl-, $C_{1-4}$-Alkoxy-, $C_{3-5}$-Cycloalkyl-, $C_{2-6}$-Alkenyl- bzw. $C_{3-5}$-Cycloalkoxyreste, für die $R_{10}$ und/oder $R_{13}$ stehen kann/können, substituiert sein kann/können, [ein] Chlor- und/oder Bromatom(e) ist/sind.

**3.** 3-[Acetyl]-1-[4'-(amino)-phenyl]-8-[chlor]-4-[methyl]-4,5-di-[hydro]-3H-2,3-benzodiazepin.

**4.** 1-[4'-(Amino)-phenyl]-8-[chlor]-4-[methyl]-3-[methylcarbamoyl]-4,5-di-[hydro]-3H-2,3-benzodiazepin.

**5.** Verfahren zur Herstellung der 2,3-Benzodiazepinderivate nach Anspruch 1 bis 4, **dadurch gekennzeichnet, daß** man in an sich bekannter Weise

a) in 7- und/oder 8-halogensubstituierte 2,3-Benzodiazepinderivate, die in der 3-Stellung nicht substituiert sind, der allgemeinen Formel

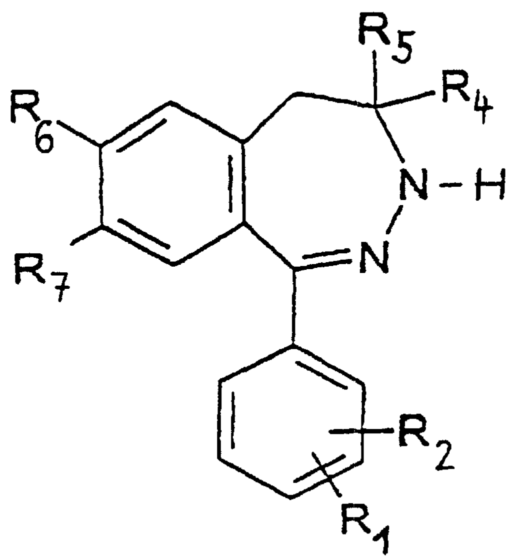

VI,

worin $R_1$ , $R_2$ , $R_4$ , $R_5$ , $R_6$ und $R_7$ wie im Anspruch 1 oder 2 festgelegt sind, bzw. ihre Säureadditionssalze in die 3-Stellung den gewünschten Substituenten $R_3$ einführt oder

b) $N_2$-substituierte [<2'-(2''-{Hydroxy}-alkyl)-4'- und/oder 5'-(halogen)-phenyl>-<gegebenenfalls substituierte phenyl>-methyliden]-hydrazinderivate der allgemeinen Formel

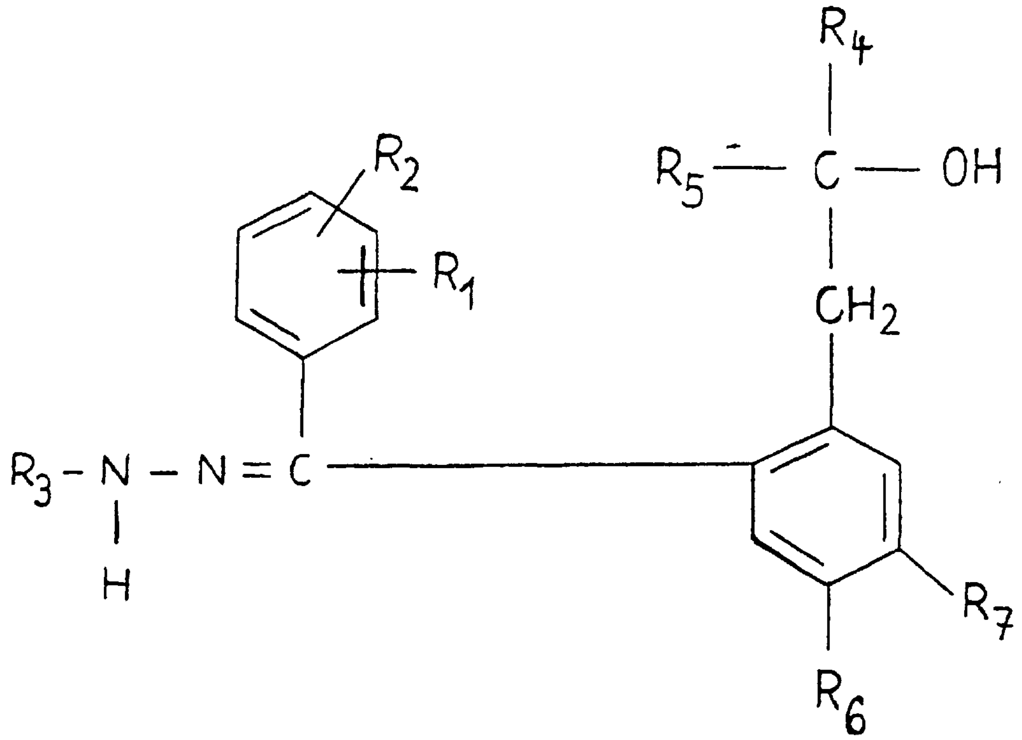

VII,

worin $R_1$, $R_2$, $R_3$, $R_4$, $R_5$, $R_6$ und $R_7$ wie im Patentanspruch 1 oder 2 festgelegt sind, mesyliert und unter alkalischen Bedingungen cyclisiert,
worauf man in an sich bekannter Weise gegebenenfalls die erhaltenen 2,3-Benzodiazepinderivate der allgemeinen Formel I in andere der allgemeinen Formel I überführt und/oder die erhaltenen 2,3-Benzodiazepinderivate der allgemeinen Formel I in Säureadditionssalze überführt oder gegebenenfalls die erhaltenen Säureadditionssalze der 2,3-Benzodiazepinderivate der allgemeinen Formel I in die entsprechenden freien Basen der allgemeinen Formel I oder in andere Salze überführt und/oder gegebenenfalls eine Trennung der erhaltenen Isomergemische der 2,3-Benzodiazepinderivate der allgemeinen Formel I beziehungsweise von deren Säureadditionssalzen in die einzelnen Isomere beziehungsweise ein Überführen einzelner Isomere in Isomergemische vornimmt.

**6.** 2,3-Benzodiazepinderivate nach Anspruch 1 bis 4 zur Verwendung als Arzneimittel.

**7.** Pharmazeutische Präparate, **gekennzeichnet durch** einen Gehalt an 1 oder mehr 2,3-Benzodiazepinderivaten nach Anspruch 1 bis 4 als Wirkstoff(en), zusammen mit 1 oder mehr üblichen pharmazeutischen Trägerstoff(en), Verdünnungsmittel(n), Lösungsmittel(n), Füllstoff(en) und/oder sonstigen Hilfsstoff(en).

**8.** Verwendung der 2,3-Benzodiazepinderivate nach Anspruch 1 bis 4 zur Herstellung von Arzneimitteln zur Behandlung von mit einem spastischen Zustand der Muskeln verbundenen Krankheiten, von Epilepsie und von akuten oder chronischen neurodegenerativen Krankheiten.

**9.** 7- und/oder 8-Halogensubstituierte 2,3-Benzodiazepinderivate, die in der 3-Stellung nicht substituiert sind, der allgemeinen Formel

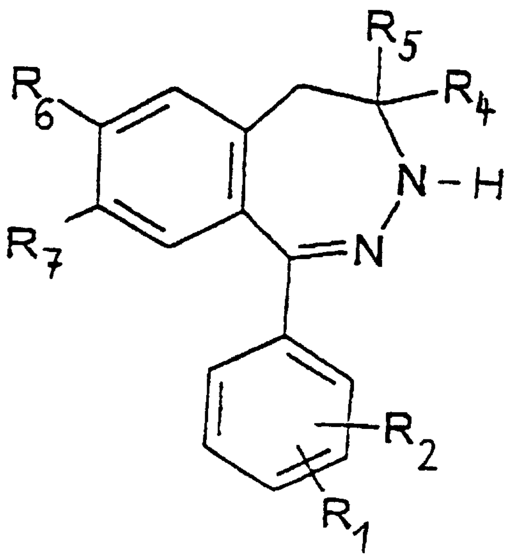

VI,

worin $R_1$ , $R_2$ , $R_4$ , $R_5$ , $R_6$ und $R_7$ wie im Anspruch 1 oder 2 festgelegt sind, sowie deren Isomere und die Säureadditionssalze dieser Verbindungen.

## Claims

**1.** 7- and/or 8-halogen-substituted 2,3-benzodiazepine derivatives of the general formula

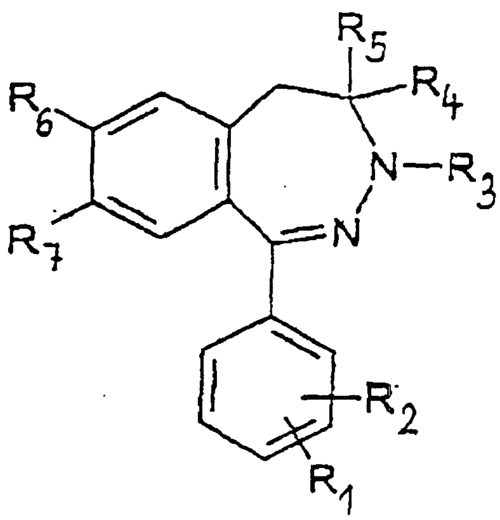

I,

wherein
$R_1$ and $R_2$ independently represent hydrogen or halogen atoms, respectively, or $C_{1-4}$-alkyl, $C_{1-4}$-alkoxy, nitro- or trifluoromethyl groups or an amino group of the general formula

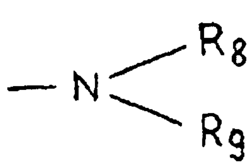

II

in the latter of which
$R_8$ and $R_9$ independently of each other are hydrogen atoms, $C_{1-4}$-alkyl groups or groups of the general formula

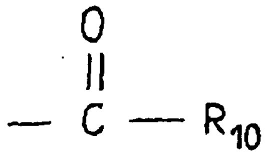

III

in the latter of which
$R_{10}$ represents a hydrogen atom or, as optionally substituted by 1 or a plurality of $C_{1-4}$-alkoxy group(s) and/or halogen atom(s), a $C_{1-6}$-alkyl, $C_{6-10}$-aryl, $C_{1-4}$-alkoxy, $C_{3-5}$-cycloalkyl, $C_{2-6}$-alkenyl or $C_{3-5}$-cycloalkoxy group or amino group of the general formula

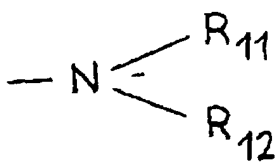

IV

in the latter of which
$R_{11}$ and $R_{12}$ are, independent of one another, hydrogen atoms or $C_{1-4}$-alkyl, $C_{3-5}$-cycloalkyl or $C_{6-10}$-aryl groups,
$R_3$ represents a $C_{1-4}$-alkyl or $C_{3-5}$-cycloalkyl group or a group ofthe general formula

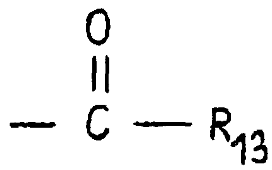

V

in the latter of which
$R_{13}$ has the same meaning as $R_{10}$,
$R_4$ and $R_5$, independent of one another, represent hydrogen or $C_{1-3}$-alkyl groups and
$R_6$ and $R_7$, independent of one another, represent hydrogen, chlorine or bromine atoms, with the further proviso that at least 1 of $R_6$ and $R_7$ is not a hydrogen,
as well as their isomers and the acid-addition salts of these compounds.

**2.** 2,3-Benzodiazepine derivatives, according to Claim 1, **characterized in that** the alkyl group(s), which can be represented by $R_1$, $R_2$, $R_8$, $R_9$, $R_{11}$, $R_{12}$ and/or $R_3$, is/are one/those having 1 to 3, particularly 1 or 2, carbon atom (s), the alkoxy group(s), which can be represented by $R_1$, $R_2$, $R_{10}$ and/or $R_{13}$ and/or through which the $C_{1-6}$-alkyl, $C_{6-10}$-aryl, $C_{1-4}$-alkoxy, $C_{3-5}$-cycloalkyl, $C_{2-6}$-alkenyl or $C_{3-5}$-cycloalkoxy groups, which in turn can be represented by $R_{10}$ and/or $R_{13}$, can be substituted, is/are one/those having 1 to 3, particularly 1 or 2, carbon atom(s), the alkyl group(s), which can be represented by $R_{10}$ and/or $R_{13}$, is/are one/those having 1 to 4, particularly 1 to 3, carbon atom(s), the alkyl group(s), which can be represented by $R_4$ and/or $R_5$, is/are one/those having 1 to 2, particularly 1, carbon atom(s), the alkenyl group(s), which can be represented by $R_{10}$ and/or $R_{13}$, is/are one/those having 2 to 4, particularly 2 or 3, carbon atom(s), the cycloalkyl or cycloalkoxy group(s), which can be represented by $R_{10}$ and/or $R_{13}$, and/or the cycloalkyl group(s), which can be represented by $R_3$, $R_{11}$ and/or $R_{12}$, is/are one/those having 3 or 4, particularly 3, carbon atom(s), the aryl group(s), which can be represented by $R_{10}$, $R_{13}$, $R_{11}$ and/or $R_{12}$, is/are [a] phenyl and/or naphthyl group(s) and/or the halogen atom(s), which can be represented by $R_1$ and/or $R_2$, and/or through which the $C_{1-6}$-alkyl, $C_{6-10}$-aryl, $C_{1-4}$-alkoxy, $C_{3-5}$-cycloalkyl, $C_{2-6}$-alkenyl or $C_{3-5}$-cycloalkoxy groups, which in turn can be represented by $R_{10}$ and/or $R_{13}$, can be substituted, is/are [a] chlorine and/or bromine atom(s).

**3.** 3-[Acetyl]-1-[4'-(amino)-phenyl]-8-[chloro]-4-[methyl]-4,5-di-[hydro]-3H-2,3-benzodiazepine.

**4.** 1-[4'-(Amino)-phenyl]-8-[chloro]-4-[methyl)-3-[methylcarbamoyl]-4,5-di-[hydro]-3H-2,3-benzodiazepine.

**5.** Process for the production of 2,3-benzodiazepine derivatives, according to Claims 1 to 4, **characterized in that**, in a *per se* known manner,

a) into 7- and/or 8-halogen substituted 2,3-benzodiazepine derivatives, which are not substituted in the 3-position, of the general formula

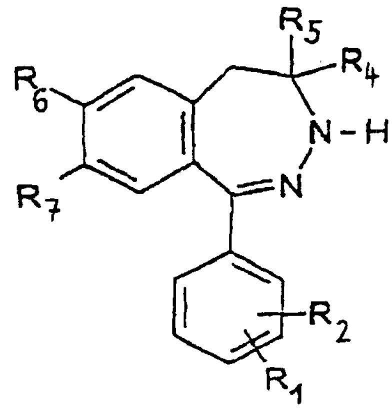

VI,

wherein $R_1$, $R_2$, $R_4$, $R_5$, $R_6$, and $R_7$ are as defined in Claim 1 or 2, or their acid-addition salts, is introduced the desired $R_3$ substituent at the 3-position
or
b) $N_2$-substituted [<2'-(2"-{hydroxy}-alkyl)-4'- and/or 5'(halogen)-phenyl>-<optionally substituted phenyl>-methyliden]-hydrazine derivatives of the general formula

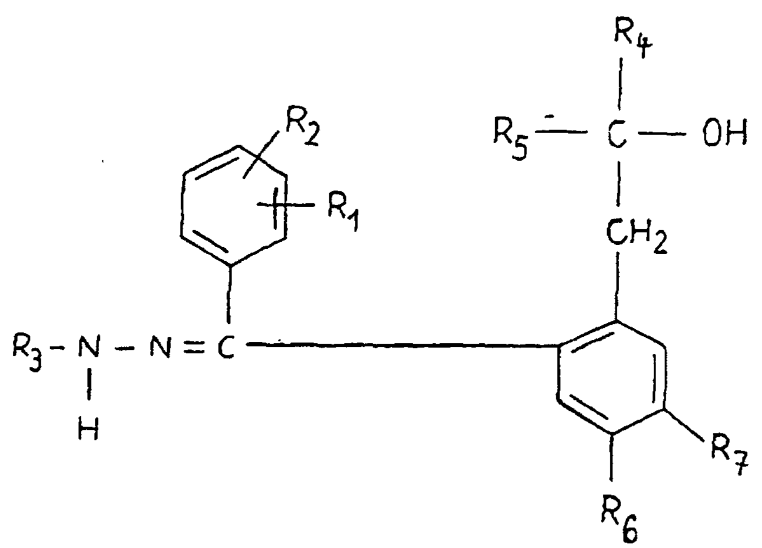

VII,

wherein $R_1$, $R_2$, $R_3$, $R_4$, $R_5$, $R_6$, and $R_7$ are as defined in Claim 1 or 2, are mesylated and cyclized under alkaline conditions,
after which, in a *per se* known manner, optionally, the obtained 2,3-benzodiazepine derivates of the general formula I are converted into others of the general formula I and/or the obtained 2,3-benzodiazepine derivates of the general formula I are converted into acid-addition salts or, optionally, the obtained acid-addition salts of the 2,3-benzodiazepine derivates of the general formula I are converted into the corresponding free bases of the general formula I or other salts and/or, optionally, a separation of the obtained isomer mixtures of the 2,3-benzodiazepine derivatives of the general formula I or, respectively, their acid-addition salts into the individual isomers or a conversion of

individual isomers into isomer mixtures is performed.

6. 2,3-Benzodiazepine derivates, according to Claims 1 to 4, for use as drugs.

7. Pharmaceutical preparations, **characterized by** having a content of 1 or a plurality of 2,3-benzodiazepine derivates, according to Claims 1 to 4, as active substance(s), together with I or a plurality of common pharmaceutical carrier substance(s), diluent(s), solvent(s), filler(s) and/or other auxiliary agent(s).

8. Use of 2,3-benzodiazepine derivatives, according to Claims 1 to 4, for the production of pharmaceuticals for the treatment of diseases involving a spastic condition of the muscles, epilepsy and acute or chronic neurodegenerative diseases.

9. 7- and/or 8-Halogen substituted 2,3-benzodiazepine derivatives, which are not substituted in the 3-position, of the general formula

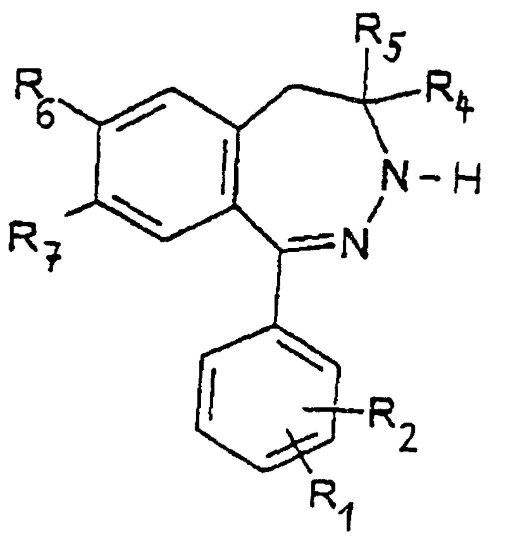

VI,

wherein $R_1$, $R_2$, $R_4$, $R_5$, $R_6$, and $R_7$ are as defined in Claim 1 or 2,
as well as their isomers and the acid-addition salts of these compounds.

**Revendications**

1. Dérivés de 2,3-benzodiazépine substitués par un halogène en position 7 et/ou 8, de formule générale

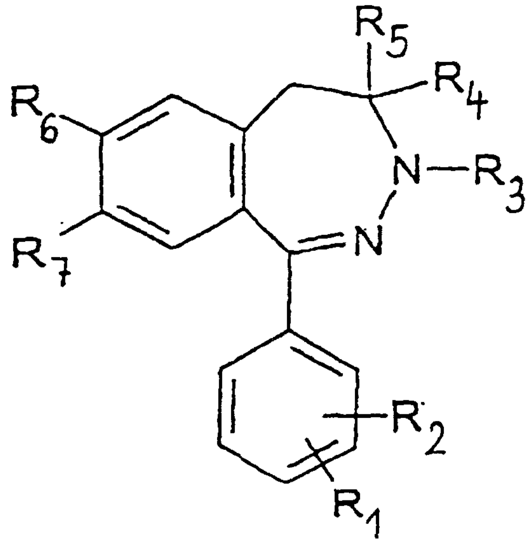

I

dans laquelle

$R_1$ et $R_2$ représentent indépendamment l'un de l'autre un atome d'hydrogène ou d'halogène ou un groupe alkyle en $C_{1-4}$, alcoxy en $C_{1-4}$, nitro ou trifluorométhyle, ou un résidu amino de formule générale

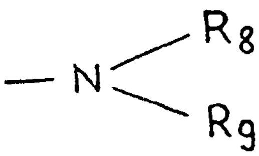

II

dans laquelle

$R_8$ et $R_9$ signifient indépendamment l'un de l'autre un atome d' hydrogène, un résidu alkyle en $C_{1-4}$ ou un résidu de formule générale

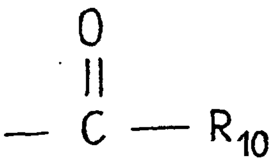

III

dans laquelle

$R_{10}$ représente un atome d'hydrogène ou un ou plus résidu alkoxy et/ou atome d'halogène substitué, un résidu alkyle en $C_{1-6}$, aryle en $C_{6-10}$, alcoxy en $C_{1-4}$, cycloalkyle en $C_{3-5}$, alcényle en $C_{2-6}$ ou cycloalcoxy en $C_{3-5}$, éventuellement substitués par un ou plusieurs résidus alcoxy en $C_{1-4}$ et/ou par un ou plusieurs atomes d'halogène, ou un résidu amino de formule générale

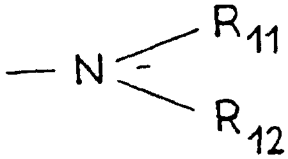

IV

dans laquelle

$R_{11}$ et $R_{12}$ sont indépendamment l'un de l'autre un atome d'hydrogène ou un résidu alkyle en $C_{1-4}$, cycloalkyle

en $C_{3-5}$ ou aryle en $C_{6-10}$,

$R_3$ signifie un résidu alkyle en $C_{1-4}$ ou cycloalkyle en $C_{3-5}$ ou un résidu de formule générale

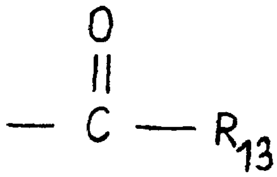

V

dans laquelle

$R_{13}$ a les mêmes significations que $R_{10}$,

$R_4$ et $R_5$ représentent indépendamment l'un de l'autre un atome d'hydrogène ou un résidu alkyle en $C_{1-3}$ et

$R_6$ et $R_7$ représentent indépendamment l'un de l'autre un atome d'hydrogène, de chlore ou de brome,

avec la condition supplémentaire qu'au moins 1 de $R_6$ et $R_7$ soit différent de l'hydrogène,

ainsi que leurs isomères et les sels d'addition d'acide de ces composés.

**2.** Dérivés de 2,3-benzodiazépine selon la revendication 1, **caractérisés en ce que** le/les résidu(s) alkyle, qui peut/peuvent être représenté(s) par $R_1$, $R_2$, $R_8$, $R_9$, $R_{11}$, $R_{12}$ et/ou $R_3$ est/sont (un) de tel (s) groupe (s) ayant de 1 à 3, en particulier 1 ou 2 atomes de carbone, le/les résidu(s) alcoxy, qui peut/peuvent signifier $R_1$, $R_2$, $R_{10}$ et/ou $R_{13}$ et/ou qui peut/peuvent être substitué(s) par les résidus alkyle en $C_{1-6}$, aryle en $C_{6-10}$, alcoxy en $C_{1-4}$, cycloalkyle en $C_{3-5}$, alcényle en $C_{2-6}$ ou résidu cycloalcoxy en $C_{3-5}$, qui peut/peuvent être représenté (s) par $R_{10}$ et/ou $R_{13}$, est/sont (un) de tel(s) groupe(s) ayant de 1 à 3, en particulier 1 ou 2 atomes de carbone, le/les résidu(s) alkyle, qui peut/peuvent être représenté (s) par $R_{10}$ et/ou $R_{13}$, est/sont (un) de tel(s) groupe(s) ayant de 1 à 4, en particulier de 1 à 3 atomes de carbone, le/les résidu(s) alkyle, qui peut/peuvent être représenté(s) par $R_4$ et/ou $R_5$, est/sont (un) de tel(s) groupe(s) ayant 1 ou 2, en particulier 1 atome de carbone, le/les résidu(s) alcényle, qui peut/peuvent être représenté (s) par $R_{10}$ et/ou $R_{13}$, est/sont (un) de tel (s) groupe (s) ayant de 2 à 4, en particulier 2 ou 3 atomes de carbone, le/les résidu(s) cycloalkyle ou cycloalcoxy, qui peut/peuvent signifier $R_{10}$ et/ou $R_{13}$ et/ou le/les résidu (s) cycloalkyle qui peut/peuvent être représenté(s) par $R_3$, $R_{11}$ et/ou $R_{12}$, est/sont (un) de tel (s) groupe(s) ayant 3 ou 4, en particulier 3 atomes de carbone, le/les résidu(s) aryle, qui peut/peuvent être représenté(s) par $R_{10}$, $R_{13}$, $R_{11}$ et/ou $R_{12}$, est/sont un ou plusieurs résidu(s) phényle et/ou naphtyle et/ou le/les atome(s) d'halogène qui peut/peuvent être représenté(s) par $R_1$ et/ou $R_2$ et/ou qui peut/peuvent être un substituant des résidus alkyle en $C_{1-6}$, aryle en $C_{6-10}$, alcoxy en $C_{1-4}$, cycloalkyle en $C_{3-5}$, alcényle en $C_{2-6}$ ou cycloalcoxy en $C_{3-5}$, qui peut/peuvent être représenté(s) par $R_{10}$ et/ou $R_{13}$, est/sont un ou plusieurs atome(s) de chlore et/ou de brome.

**3.** 3-[acétyl]-1-[4'-(amino)-phényl]-8-[chloro]-4-[méthyl]-4,5-di-[hydro]-3H-2,3-benzodiazépine.

**4.** 1-[4'-(amino)-phényl]-8-[chloro]-4-[méthyl]-3-[méthylcarbamoyl]-4,5-di-[hydro]-3H-2,3-benzodiazépine.

**5.** Procédé de préparation des dérivés de 2,3-benzodiazépine selon l'une des revendications 1 à 4, **caractérisé en ce que**, d'une manière connue en soi :

a) on introduit le substituant $R_3$ désiré en position 3 dans les dérivés de 2,3-benzodiazépine substitués par un halogène en position 7 et/ou 8, qui ne sont pas substitués en position 3, de formule générale

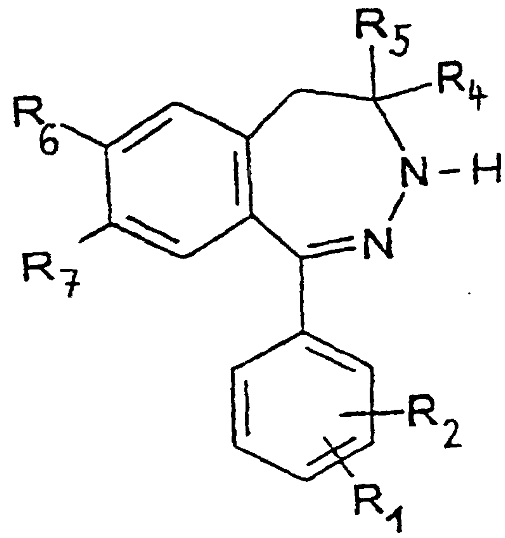

VI

dans laquelle $R_1$, $R_2$, $R_4$, $R_5$, $R_6$ et $R_7$ sont définis comme dans la revendication 1 ou 2, ou dans leurs sels d'addition d'acide

ou

b) des dérivés de [<2'-(2"-{hydroxy}-alkyl)-4'- et/ou 5'-(halogéno)-phényl>-<éventuellement à substitution phényle>-méthylidène]-hydrazine $N_2$-substitués de formule générale

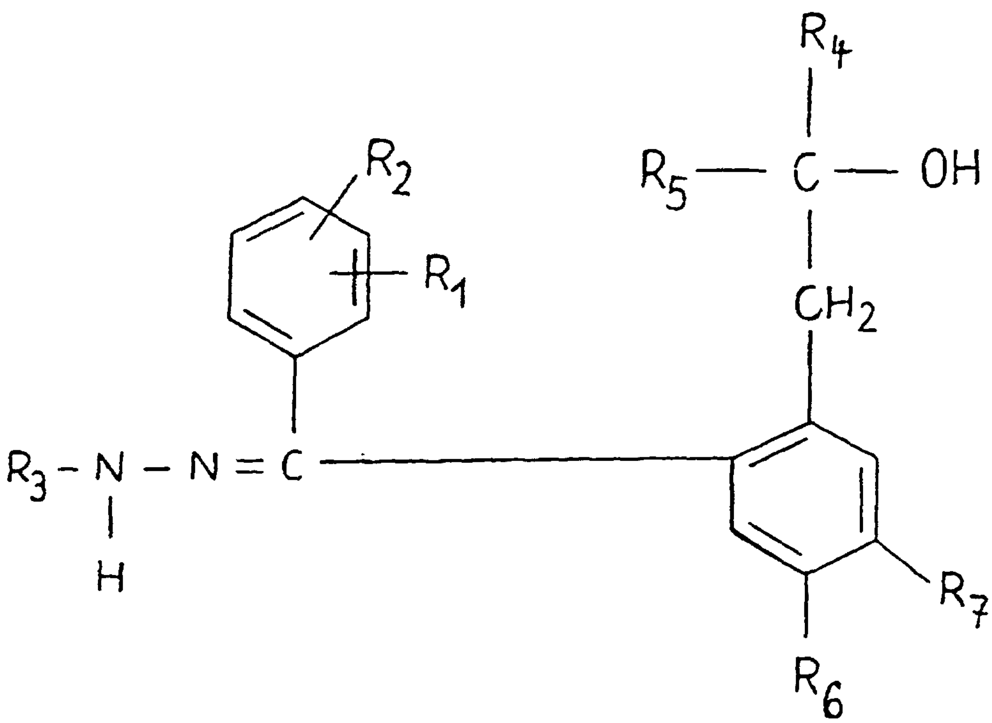

VII

dans laquelle $R_1$, $R_2$, $R_3$, $R_4$, $R_5$, $R_6$ et $R_7$ sont définis comme dans la revendication 1 ou 2, sont mésylés et cyclisés dans des conditions alcalines,

après quoi, d'une manière connue en soi, le cas échéant, on transforme les dérivés obtenus de 2,3-benzodiazépine de formule générale I en d'autres dérivés de formule générale I, et/ou on transforme les dérivés obtenus de 2,3-benzodiazépine de formule générale I en des sels d'addition d'acide ou, le cas échéant, on transforme les sels d'addition d'acide obtenus des dérivés de 2,3-benzodiazépine de formule générale I en les bases libres correspondantes de formule générale I et/ou, le cas échéant, on effectue une séparation des mélanges d'isomères

obtenus des dérivés de 2,3-benzodiazépine de formule générale I ou de leurs sels d'addition d'acide en les isomères individuels ou on effectue une transformation des isomères individuels en des mélanges d'isomères.

**6.** Dérivés de 2,3-benzodiazépine selon l'une des revendications 1 à 4 à utiliser en tant que médicament.

**7.** Préparations pharmaceutiques, **caractérisées par** une teneur en 1 ou plusieurs dérivés de 2,3-benzodiazépine selon l'une des revendications 1 à 4 en tant que principe (s) actif (s) , conjointement avec un ou plusieurs véhicule (s), diluant(s), solvant(s), charge(s) et/ou autre(s) auxiliaire(s) pharmaceutique(s) usuel(s).

**8.** Utilisation des dérivés de 2,3-benzodiazépine selon l'une des revendications 1 à 4 pour la fabrication de médicaments pour le traitement de maladies liées à un état spastique des muscles, de l'épilepsie et de maladies neurodégénérescentes aiguës ou chroniques.

**9.** Dérivés de 2,3-benzodiazépine substitués par un halogène en position 7 et/ou 8, qui ne sont pas substitués en position 3, répondant à la formule générale

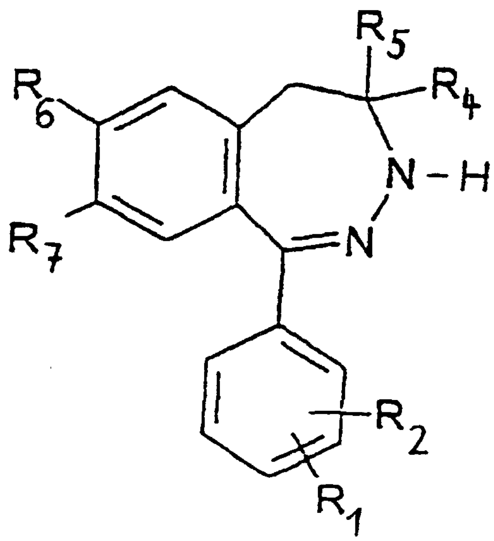

VI

dans laquelle $R_1$, $R_2$, $R_4$, $R_5$, $R_6$ et $R_7$ sont définis comme dans la revendication 1 ou 2, ainsi que leurs isomères et les sels d'addition d'acide de ces composés.